(19) Europäisches Patentamt
European Patent Office
Office européen des brevets

(11) EP 3 475 253 B1

(12) **EUROPÄISCHE PATENTSCHRIFT**

(45) Veröffentlichungstag und Bekanntmachung des Hinweises auf die Patenterteilung:
**30.09.2020 Patentblatt 2020/40**

(21) Anmeldenummer: **17733421.6**

(22) Anmeldetag: **22.06.2017**

(51) Int Cl.:
***C07C 51/235*** (2006.01) ***C07C 59/125*** (2006.01)

(86) Internationale Anmeldenummer:
**PCT/EP2017/065413**

(87) Internationale Veröffentlichungsnummer:
**WO 2018/001861 (04.01.2018 Gazette 2018/01)**

(54) **VERFAHREN ZUR HERSTELLUNG VON 2-METHOXYESSIGSÄURE**

METHOD FOR PRODUCING 2-METHOXYACETIC ACID

PROCÉDÉ DE PRÉPARATION D'ACIDE 2-MÉTHOXYACÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.06.2016 EP 16176637**

(43) Veröffentlichungstag der Anmeldung:
**01.05.2019 Patentblatt 2019/18**

(73) Patentinhaber: **BASF SE**
**67056 Ludwigshafen am Rhein (DE)**

(72) Erfinder:
- **VAUTRAVERS, Nicolas**
**67056 Ludwigshafen (FR)**
- **TELES, Joaquim Henrique**
**67056 Ludwigshafen (DE)**
- **POTTGIESSER, Werner**
**67056 Ludwigshafen (DE)**
- **ALTHOEFER, Henning**
**67056 Ludwigshafen (DE)**

(74) Vertreter: **BASF IP Association**
**BASF SE**
**G-FLP-C006**
**67056 Ludwigshafen (DE)**

(56) Entgegenhaltungen:
**DE-A1- 2 936 123** **DE-A1- 3 135 946**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Herstellung von 2-Methoxyessigsäure durch Oxidation von 2-Methoxyethanol in einer Reaktionsvorrichtung mit Sauerstoff bei einer Temperatur von 20 bis 100°C und einem Sauerstoff-Partialdruck von 0,01 bis 2 MPa in Gegenwart von Wasser und eines heterogenen Katalysators enthaltend Platin.

2-Methoxyessigsäure ist ein wichtiges Zwischenprodukt in der chemischen Synthese. Durch Halogenierung mit Phosgen oder Thionylchlorid kann daraus etwa 2-Methoxyessigsäurechlorid als reaktiver Synthesebaustein gewonnen werden. Dieser findet etwa in der Synthese der fungiziden Wirkstoffe Metalaxyl und Oxadixyl Anwendung.

2-Methoxyessigsäure ist technisch beispielsweise durch katalytische Oxidation von 2-Methoxyethanol zugänglich.

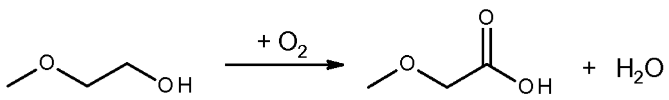


US 3,342,858 beschreibt allgemein die Herstellung von Alkoxyessigsäuren durch Oxidation der entsprechenden Alkoxyethanole mit Sauerstoff in Gegenwart von Wasser, einer Base, wie beispielsweise Natriumhydroxid, und eines Platin enthaltenden Katalysators bei einem pH-Wert von > 7 unter Bildung des entsprechenden Alkoxyacetat-Salzes, anschließender Freisetzung der Alkoxyessigsäure durch Zugabe einer Säure, wie beispielsweise Salzsäure, und destillativer Gewinnung der Alkoxyessigsäure aus dem angesäuertem Reaktionsgemisch.

Nachteilig an diesem Verfahren ist das sehr aufwendige Reaktionskonzept unter Bildung des entsprechenden Alkoxyacetat-Salzes als Zwischenprodukt und anschließender Freisetzung der Alkoxyessigsäure in einem Folgeschritt. Diese Vorgehensweise erfordert die Zugabe einer Base und nachfolgend einer Säure als Hilfsstoffe in jeweils stöchiometrischer Größenordnung. Das als Zwangsanfall gebildete Salz aus der zugegebenen Base und zugegebenen Säure ist aufwendig zu entsorgen. Zudem bedarf es zur Isolierung der Alkoxyessigsäure einer fraktionierten Destillation. Die Ausbeute an Alkoxyessigsäure beträgt lediglich 50 bis 90%.

DE 29 36 123 A lehrt die Herstellung von Alkoxyessigsäuren durch Oxidation der entsprechenden Alkoxyethanole mit Sauerstoff in Gegenwart von Wasser und eines Platin enthaltenden Katalysators bei einem pH-Wert von ≤ 7 unter direkter Bildung der Alkoxyessigsäure im Reaktionsgemisch. Die Beispiele in DE 29 36 123 A betreffen sowohl die diskontinuierliche als auch die kontinuierliche Reaktionsführung.

Bei der diskontinuierlichen Reaktionsführung in Beispiel 1 der DE-Schrift wurde eine 15%-ige, wässrige 2-Methoxyethanol-Lösung sowie ein Katalysator mit 5% Pt auf Aktivkohle in einem Glasrohr vorgelegt und bei Normaldruck und 45°C Sauerstoff hindurchgeleitet. Hierdurch wurde eine Ausbeute an 2-Methoxyessigsäure von 95% erreicht.

Bei der kontinuierlichen Reaktionsführung in Beispiel 4 der DE-Schrift wurde über mehrere Wochen hinweg bei 0,5 MPa und 48 bis 53°C ein konstanter Strom einer 20%-ige, wässrige 2-Methoxyethanol-Lösung zusammen mit Sauerstoff durch ein Edelstahlrohr geleitet, in dem sich ein Katalysator mit 10% Pt auf Aktivkohle befand. Der kondensierbare Anteil des Reaktionsgemisches wurde analysiert und eine Ausbeute an 2-Methoxyessigsäure bezogen auf das umgesetzte 2-Methoxyethanol von über 90% nachgewiesen. Dies entspricht rechnerisch der Bildung von bis zu 10% an Nebenprodukten bezogen auf das umgesetzte 2-Methoxyethanol.

Nachteilig an diesem Verfahren ist der offensichtlich hohe Anteil an Nebenprodukten, der zum einen einen Verlust an Edukt darstellt und zum anderen eine aufwendige Aufarbeitung des Reaktionsgemisches erfordert.

CN 104892390 A offenbart ebenfalls die Herstellung von 2-Methoxyessigsäure durch Oxidation von 2-Methoxyethanol mit Sauerstoff in Gegenwart von Wasser und Pt/C als Katalysator. Die Beispiele 1 bis 5 beschreiben die diskontinuierliche Verfahrensführung, wobei jeweils der Pt/C-Katalysator und eine wässrigen 2-Methoxyethanol Lösung mit einer Konzentration von 2-Methoxyethanol im Bereich von 32,4 bis 49,0 Gew.-% in einem Reaktor vorgelegt und anschließend mit Sauerstoff unter Variation von Druck, Temperatur und Reaktionszeit umgesetzt wurde. Das erhaltene Reaktionsgemisch wurde jeweils im Vakuum zur Isolierung der 2-Methoxyessigsäure destilliert. Es wurde jeweils eine Reinheit von 99% erhalten. Die Ausbeute lag im Bereich 91 bis 96%.

Nachteilig an diesem Verfahren ist die erforderliche destillative Abtrennung der 2-Methoxyessigsäure um wenigstens eine Reinheit von 99% zu erhalten.

Erfindungsgemäß wurde erkannt, dass die wesentliche Nebenreaktion bei der katalytischen Oxidation von 2-Methoxyethanol zur 2-Methoxyessigsäure die Bildung von Methoxyessigsäure-2-methoxyethylester ist.

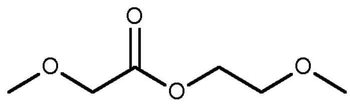

Methoxyessigsäure-2-methoxyethylester

So wurde im Rahmen der vorliegenden Erfindung festgestellt, dass bei den Verfahren nach dem Stand der Technik 2-Methoxyethanol mit einem relativ hohen Gehalt an Methoxyessigsäure-2-methoxyethylester erhalten wird. In einem experimentellen Vergleichsbeispiel in diskontinuierlicher Fahrweise, bei dem die gesamte Menge an 2-Methoxyethanol im Reaktor vorgelegt wurde, wurde beispielsweise ein 2-Methoxyethanol-Austrag mit einem Gehalt von 2,6 Gew.-% Methoxyessigsäure-2-methoxyethylester erhalten (Vergleichsbeispiel 1).

Je nach Verwendung der 2-Methoxyessigsäure stören jedoch bereits Restgehalte an Methoxyessigsäure-2-methoxyethylester im unteren Prozentbereich. Es besteht daher großes Interesse an der Gewinnung von 2-Methoxyessigsäure mit einem Gehalt an Methoxyessigsäure-2-methoxyethylester von ≤ 1,5 Gew.-%.

Aufgrund der Lage der Siedepunkte ist es zwar möglich, Wasser (Siedepunkt 46°C bei 100 hPa) und nicht umgesetztes 2-Methoxyethanol (Siedepunkt 54°C bei 100 hPa) relativ leicht von 2-Methoxyessigsäure (Siedepunkt 125°C bei 100 hPa) abzutrennen, nicht jedoch Methoxyessigsäure-2-methoxyethylester (Siedepunkt 123°C bei 100 hPa), da dieser nur knapp unterhalb der 2-Methoxyessigsäure siedet. Eine destillative Abtrennung des Methoxyessigsäure-2-methoxyethylesters von der 2-Methoxyessigsäure wäre extrem aufwendig und würde eine Destillationskolonne mit einer sehr großen Anzahl theoretischer Böden sowie die Einstellung eines hohes Rücklaufverhältnisses erfordern. Zudem wäre der Betrieb einer derartigen Kolonne relativ energieintensiv. Außerdem wäre trotz einer sehr großen Anzahl theoretischer Böden und eines hohes Rücklaufverhältnisses auch mit einem Verlust an 2-Methoxyessigsäure-Zielprodukt zu rechnen. Eine nicht zu unterschätzende negative Eigenschaft von 2-Methoxyessigsäure ist deren Korrosivität in Verbindung mit Wasser. So müsste auch die Reinigungskolonne aus einem korrosionsfesten Werkstoff bestehen, was einen hohen Aufwand in der Bereitstellung der Kolonne darstellt. Nicht zuletzt stellt der gebildete Methoxyessigsäure-2-methoxyethylester auch einen direkten Verlust an 2-Methoxyethanol-Einsatzstoff und 2-Methoxyessigsäure-Zielprodukt dar.

Auch in DE 33 45 807 A wurde die Problematik einer destillativen Gewinnung von 2-Methoxyessigsäure aus einem 2-Methoxyessigsäure und Methoxyessigsäure-2-methoxyethyl-ester enthaltendem Gemisch bereits erkannt. Als Lösung schlägt die DE-Schrift ein gänzlich anderes Trennverfahren vor, nämlich die langsame Auskristallisation von 2-Methoxyessigsäure bei einer Temperatur unterhalb von 8,5°C. 2-Methoxyessigsäure kann dadurch auch aus einer Lösung mit 4 Gew.-% Methoxyessigsäure-2-methoxyethylester in einer Reinheit von 99,8 Gew.-% gewonnen werden. Methoxyessigsäure-2-methoxyethylester verbleibt in der Mutterlauge.

Das in DE 33 45 807 A vorgeschlagene Verfahren ermöglicht zwar die Gewinnung von 2-Methoxyessigsäure in hoher Reinheit, erfordert jedoch einen zusätzlichen Verfahrensschritt in einem Kristallisationsapparat. Neben der Bereitstellung des Kristallisationsapparats und dem Aufwand zur Durchführung der Kristallisation erfordert dieses Verfahren auch die energieintensive Bereitstellung eines Kühlmediums zur Abkühlung der gesamten Lösung auf eine Temperatur unterhalb von 8,5°C. Das Verfahren ist somit relativ aufwendig durchzuführen und energieintensiv.

Es war daher die Aufgabe der vorliegenden Erfindung, ein Verfahren zur Herstellung von 2-Methoxyessigsäure zu finden, welches die Nachteile des Standes der Technik vermeidet, auf leicht zugänglichen Einsatzstoffen beruht, einfach durchzuführen ist, 2-Methoxyessigsäure in möglichst hoher Selektivität, Ausbeute und Reinheit bildet und insbesondere das Nebenprodukt Methoxyessigsäure-2-methoxyethylester in deutlich geringerer Menge erzeugt als in den Verfahren nach dem Stand der Technik. Ferner soll das Verfahren trotz einer im Vergleich zum Stand der Technik geringeren Bildung von Methoxyessigsäure-2-methoxyethylester und somit höheren Selektivität zur 2-Methoxyessigsäure mit einem möglichst kleinen Reaktionsvolumen auskommen und somit idealerweise ein Reaktionsgemisch mit einer möglichst hohen Konzentration an 2-Methoxyessigsäure zur Verfügung stellen. Des Weiteren soll das Verfahren eine möglichst einfache Aufarbeitung des Reaktionsgemisches ermöglichen.

Überraschend wurde ein Verfahren zur Herstellung von 2-Methoxyessigsäure durch Oxidation von 2-Methoxyethanol in einer Reaktionsvorrichtung mit Sauerstoff bei einer Temperatur von 20 bis 100°C und einem Sauerstoff-Partialdruck von 0,01 bis 2 MPa in Gegenwart von Wasser und eines heterogenen Katalysators enthaltend Platin gefunden, bei dem man das Verfahren halbkontinuierlich oder kontinuierlich durchführt, und man die Zugabe von 2-Methoxyethanol zur Reaktionsvorrichtung zeitlich und räumlich so wählt, dass in der 2-Methoxyethanol und 2-Methoxyessigsäure enthaltenden Flüssigphase in der Reaktionsvorrichtung der Quotient CR/CA zeitlich und räumlich stets ≤ 0,80 beträgt,

wobei CR definiert ist als

$$CR = \frac{C(2Methoxyethanol\ Reaktor)}{C(2Methoxyethanol\ Reaktor)+C(Wasser\ Reaktor)},$$

und darin C(2Methoxyethanol Reaktor) für die Masse an 2-Methoxyethanol pro Volumenelement der 2-Methoxyethanol und 2-Methoxyessigsäure enthaltenden Flüssigphase, und C(Wasser Reaktor) für die Masse an Wasser pro Volumenelement der 2-Methoxyethanol und 2-Methoxyessigsäure enthaltenden Flüssigphase steht,
beim halbkontinuierlichen Verfahren CA definiert ist als

$$CA = \frac{MT(2Methoxyethanol\ Gesamtmasse)}{MT(2Methoxyethanol\ Gesamtmasse)+MT(Wasser\ Gesamtmasse)},$$

wobei MT(2Methoxyethanol Gesamtmasse) für die insgesamt beim halbkontinuierlichen Verfahren eingesetzte Masse an 2-Methoxyethanol, und MT(Wasser Gesamtmasse) für die insgesamt beim halbkontinuierlichen Verfahren eingesetzte Masse an Wasser steht, und
beim kontinuierlichen Verfahren CA definiert ist als

$$CA = \frac{MF(2Methoxyethanol\ Massenstrom)}{MF(2Methoxyethanol\ Massenstrom)+MF(Wasser\ Massenstrom)},$$

wobei MF(2Methoxyethanol Massenstrom) für den, der Reaktionsvorrichtung zugeführten Massenstrom an 2-Methoxyethanol, und MF(Wasser Massenstrom) für den, der Reaktionsvorrichtung zugeführten Massenstrom an Wasser steht.

Gegenüber den bekannten Verfahren nach dem Stand der Technik führt das erfindungsgemäße Verfahren bei gleicher Zugabemenge an 2-Methoxyethanol und gleichem Reaktorvolumen zu einem Reaktionsgemisch mit einer höheren Reinheit an 2-Methoxyessigsäure und insbesondere einem deutlich geringeren Gehalt an unerwünschtem Methoxyessigsäure-2-methoxyethylester. Dieses überraschende Ergebnis wird erreicht durch die erfindungsgemäße Zugabe von 2-Methoxyethanol, bei der das 2-Methoxyethanol der Reaktionsvorrichtung zeitlich und räumlich so zugegeben wird, dass in der 2-Methoxyethanol und 2-Methoxyessigsäure enthaltenden Flüssigphase in der Reaktionsvorrichtung der Quotient CR/CA zeitlich und räumlich stets ≤ 0,80 beträgt.

Unter Reaktionsvorrichtung ist die apparative Einheit zu verstehen, welche den Reaktor oder gegebenenfalls mehrere miteinander verbundener Reaktoren einschließlich erforderlicher Wärmetauschervorrichtungen zur Kühlung oder Heizung und etwaiger Vorrichtungen zur partiellen Rückführung des Reaktionsgemisches enthält.

Der Wert CR ist definiert als

$$CR = \frac{C(2Methoxyethanol\ Reaktor)}{C(2Methoxyethanol\ Reaktor)+C(Wasser\ Reaktor)},$$

wobei C(2Methoxyethanol Reaktor) für die Masse an 2-Methoxyethanol pro Volumenelement der 2-Methoxyethanol und 2-Methoxyessigsäure enthaltenden Flüssigphase, und C(Wasser Reaktor) für die Masse an Wasser pro Volumenelement der 2-Methoxyethanol und 2-Methoxyessigsäure enthaltenden Flüssigphase steht. Bei dem Wert CR handelt es sich somit um die Konzentration an 2-Methoxyethanol in der 2-Methoxyethanol und 2-Methoxyessigsäure enthaltenden Flüssigphase in der Reaktionsvorrichtung, bezogen auf die Konzentration an 2-Methoxyethanol und Wasser in der Reaktionsvorrichtung. CR entspricht damit der Masse an 2-Methoxyethanol bezogen auf die Masse an 2-Methoxyethanol und Wasser. Der Wert CR ist daher grundsätzlich abhängig von Zeit und Ort. Je nach Reaktionsfortschritt ändern sich die jeweiligen Konzentrationen C(2Methoxyethanol Reaktor) und C(Wasser Reaktor) in Abhängigkeit von der Reaktionszeit sowie dem Ort in der Reaktionsvorrichtung und somit auch der Wert CR.

Beim Wert CA ist zu unterscheiden, ob das Verfahren halbkontinuierlich oder kontinuierlich durchgeführt wird.

Charakteristisch für die halbkontinuierliche Durchführung ist die Zugabe einer bestimmten Menge an 2-Methoxyethanol zur Reaktionsvorrichtung während der Reaktion, wobei in diesem Zeitraum kein Reaktionsgemisch aus der Reaktionsvorrichtung entnommen wird. Es kann sowohl die gesamte Menge an 2-Methoxyethanol während der Reaktion zugegeben werden, also auch eine gewisse Teilmenge zu Beginn vorgelegt und nur die restliche Menge während der Reaktion zugegeben werden. Hinsichtlich des Wassers ist es möglich, die Gesamtmenge zu Beginn in der Reaktionsvorrichtung vorzulegen oder mindestens einen Teil davon, wobei der verbleibende Teil dann während der Reaktion zugeführt wird. Bei der Durchführung des halbkontinuierlichen Verfahrens steigt die Flüssigkeitsmenge in der Reaktionsvorrichtung aufgrund der Zugabe von 2-Methoxyethanol sowie gegebenenfalls von Wasser an. Das Reaktionsgemisch kann dann anschließend aus der Reaktionsvorrichtung entnommen werden. Das halbkontinuierliche Verfahren wird daher in einzelnen Chargen, oder auch Batches genannt, durchgeführt. Für das halbkontinuierliche Verfahren ist CA definiert als

$$CA = \frac{MT(2Methoxyethanol\ Gesamtmasse)}{MT(2Methoxyethanol\ Gesamtmasse) + MT(Wasser\ Gesamtmasse)},$$

wobei MT(2Methoxyethanol Gesamtmasse) für die insgesamt beim halbkontinuierlichen Verfahren eingesetzte Masse an 2-Methoxyethanol, und MT(Wasser Gesamtmasse) für die insgesamt beim halbkontinuierlichen Verfahren eingesetzte Masse an Wasser steht. Bei dem Wert CA handelt es sich beim halbkontinuierlichen Verfahren also um die insgesamt bei der jeweiligen Charge eingesetzte Masse an 2-Methoxyethanol, bezogen auf die insgesamt bei der jeweiligen Charge eingesetzte Masse an 2-Methoxyethanol und Wasser. Zur insgesamt eingesetzten Masse an 2-Methoxyethanol zählt natürlich auch die Masse an eventuell im Reaktor vorgelegtem 2-Methoxyethanol. Hinsichtlich des Wassers ist sowohl die Masse an vorgelegtem Wasser als auch die Masse an eventuell zugegebenen Wasser zu berücksichtigen, nicht jedoch die Masse an gebildetem Reaktionswasser.

Charakteristisch für die kontinuierliche Durchführung ist die im zeitlichen Mittel kontinuierliche Zugabe von 2-Methoxyethanol und Wasser zur Reaktionsvorrichtung und die im zeitlichen Mittel kontinuierliche Entnahme von Reaktionsgemisch aus der Reaktionsvorrichtung. Für das kontinuierliche Verfahren ist CA definiert als

$$CA = \frac{MF(2Methoxyethanol\ Massenstrom)}{MF(2Methoxyethanol\ Massenstrom) + MF(Wasser\ Massenstrom)},$$

wobei MF(2Methoxyethanol Massenstrom) für den, der Reaktionsvorrichtung zugeführten Massenstrom an 2-Methoxyethanol, und MF(Wasser Massenstrom) für den, der Reaktionsvorrichtung zugeführten Massenstrom an Wasser steht. Da die Reaktionsvorrichtung als apparative Einheit zu verstehen ist, welche bereits etwaige Vorrichtungen zur partiellen Rückführung des Reaktionsgemisches enthält, ist ein eventuell über eine partielle Rückführung des Reaktionsgemisches rückgeführter Strom an 2-Methoxyethanol und/oder Wasser daher weder in MF(2Methoxyethanol Massenstrom) noch in MF(Wasser Massenstrom) enthalten. Bei dem Wert CA handelt es sich beim kontinuierlichen Verfahren also um die pro Zeiteinheit der Reaktionsvorrichtung neu zugeführte Masse an 2-Methoxyethanol, bezogen auf die pro Zeiteinheit der Reaktionsvorrichtung neu zugeführte Masse an 2-Methoxyethanol und Wasser. Ebenfalls unberücksichtigt in der Berechnung von CA ist das während der Reaktion gebildete Wasser.

Wesentlich für den Erfolg des erfindungsgemäßen Verfahrens ist die entsprechende zeitliche und räumliche Zugabe von 2-Methoxyethanol zur Reaktionsvorrichtung. "Zeitlich" bezieht sich auf die zeitliche Verteilung der Zugabe während der Reaktion und "räumlich" auf den Zugabeort in der Reaktionsvorrichtung. Die Formulierung "stets" macht deutlich, dass die genannte Obergrenze des Quotienten CR/CA in der 2-Methoxyethanol und 2-Methoxyessigsäure enthaltenden Flüssigphase in der Reaktionsvorrichtung an jedem Ort - also räumlich - und während der gesamten Durchführung des Verfahrens - also auch zeitlich - einzuhalten ist.

Eine zeitlich verteilte Zugabe kann beispielsweise derart erfolgen, dass eine bestimmte Menge an 2-Methoxyethanol über einen bestimmten Zeitraum der Reaktionsvorrichtung zugeführt wird. Die Zufuhr kann dabei beispielsweise über diesen Zeitraum hinweg kontinuierlich oder intermittierend erfolgen. Ebenso möglich ist beispielsweise auch eine über den Zeitraum zunehmende oder abnehmende Menge pro Zeiteinheit, wobei sich zunehmende und abnehmende Mengen natürlich auch abwechseln können.

Eine zeitlich verteilte Zugabe erfolgt bevorzugt beim halbkontinuierlichen Verfahren. Durch die Verteilung der Zugabe auf einen bestimmten Zeitraum und die teilweise chemische Umwandlung von bislang zugegebenem 2-Methoxyethanol ist es möglich, den Quotienten CR/CA in der 2-Methoxyethanol und 2-Methoxyessigsäure enthaltenden Flüssigphase in der Reaktionsvorrichtung über den Reaktionsverlauf hinweg auf einen Wert von $\leq 0{,}80$ zu halten. Im Vergleich dazu würde bei einem diskontinuierlichen Verfahren nach dem Stand der Technik unter vollständiger Zugabe der gesamten Menge an 2-Methoxyethanol zu Beginn der Reaktion der Quotienten CR/CA rechnerisch 1 betragen.

Eine räumlich verteilte Zugabe kann beispielsweise derart erfolgen, dass 2-Methoxyethanol an einem bestimmten Ort der Reaktionsvorrichtung oder verteilt an verschiedenen Orten der Reaktionsvorrichtung zugeführt wird.

Eine räumlich verteilte Zugabe erfolgt bevorzugt beim kontinuierlichen Verfahren. Durch die Verteilung der kontinuierlichen Zugabe über verschiedene Zugabeorte entlang des räumlichen Reaktionsverlaufs und die teilweise chemische Umwandlung von bereits vor der jeweiligen Zugabestelle zugegebenem 2-Methoxyethanol ist es möglich, den Quotienten CR/CA in der 2-Methoxyethanol und 2-Methoxyessigsäure enthaltenden Flüssigphase in der Reaktionsvorrichtung über den Reaktionsverlauf hinweg auf einen Wert von $\leq 0{,}80$ zu halten. Im Vergleich dazu würde bei einem kontinuierlichen Verfahren nach dem Stand der Technik im geraden Durchgang, bei dem 2-Methoxyethanol nur

an einer Stelle zugegeben wird, am räumlichen Anfang des Reaktionsverlaufs, also der Stelle, an dem sich erstmals 2-Methoxyessigsäure bildet, der Quotienten CR/CA rechnerisch 1 betragen.

Der zeitliche und räumliche Verlauf von CR kann sowohl bei der halbkontinuierlichen als auch bei der kontinuierlichen Verfahrensdurchführung beispielsweise durch einfache Versuche vorab experimentell ermittelt oder bei bekannter Reaktionskinetik vorab berechnet werden. Zur experimentellen Ermittlung kann beispielsweise die Konzentration an 2-Methoxyethanol und Wasser an verschiedenen Orten und zu verschiedenen Zeiten über den Reaktionsverlauf hinweg in der Reaktionsvorrichtung analytisch ermittelt werden. Die quantitative Analyse kann sowohl am gewünschten Ort online als auch durch Probennahme und nachfolgender offline Analytik erfolgen. 2-Methoxyethanol, 2-Methoxyessigsäure und Wasser können beispielsweise durch Gaschromatografie, IR-Spektroskopie, NIR-Spektroskopie oder $^1$H-NMR-Spektroskopie quantitativ bestimmt werden. Für die quantitative Bestimmung von 2-Methoxyessigsäure kommen auch die Leitfähigkeitsmessung, die Messung der Dielektrizitätskonstante sowie die Messung der Impedanz in Frage. Die online Analytik ermöglicht eine Verfolgung des aktuellen Reaktionsablaufs und dadurch auch eine manuelle oder automatische Anpassung der Reaktionsbedingungen während der Reaktion, wie etwa Temperatur, Sauerstoff-Partialdruck oder der Zugabemenge an 2-Methoxyethanol pro Zeiteinheit und Ort. Für die online Analytik eignen sich insbesondere die IR- und NIR-Spektroskopie, die Messung der Leitfähigkeitsmessung, der Dielektrizitätskonstante sowie der Impedanz. Zur Anpassung der Reaktionsbedingungen während der Reaktion ist es jedoch nicht zwingend erforderlich, jeweils die Konzentrationen an 2-Methoxyethanol, 2-Methoxyessigsäure und Wasser zu bestimmen. Es genügt hierzu vielfach auch die online Messung eines charakteristischen Meßwerts, wie beispielsweise der elektrischen Leitfähigkeit, der Dielektrizitätskonstante oder der Impedanz, welcher dann indirekt ein Maß für den Reaktionsverlauf darstellt. Dieser Meßwert kann dann zum Beispiel einen automatischen Regelkreis ansteuern, welcher die Zugabe an 2-Methoxyethanol regelt. Eine weitere, indirekte Verfolgung des Reaktionsverlaufs ist auch über die Messung der produzierten Wärme möglich. Die sogenannte Kalorimetrie oder Wärmebilanz kann beispielsweise vorteilhaft in der Verfolgung des halbkontinuierlichen Verfahrens eingesetzt werden.

Der Wert CA ist indes beim halbkontinuierlichen Verfahren durch die insgesamt zuzugebende Menge an 2-Methoxyethanol und Wasser, und beim kontinuierlichen Verfahren durch den zuzugebenden Massenstrom an 2-Methoxyethanol und Wasser vorgegeben.

Bevorzugt wählt man beim erfindungsgemäßen Verfahren die Zugabe von 2-Methoxyethanol zur Reaktionsvorrichtung zeitlich und räumlich derart, dass in der 2-Methoxyethanol und 2-Methoxyessigsäure enthaltenden Flüssigphase in der Reaktionsvorrichtung der Quotient CR/CA zeitlich und räumlich stets $\leq$ 0,75, besonders bevorzugt stets $\leq$ 0,70 und ganz besonders bevorzugt stets $\leq$ 0,60 beträgt. Je niedriger der Quotient CR/CA über den zeitlichen und räumlichen Verlauf der Reaktion ist, desto weniger Methoxyessigsäure-2-methoxyethylester wird in der Regel gebildet.

Um jedoch eine möglichst hohe Raum-Zeit-Ausbeute zu erhalten, ist es in der Gesamtschau von Vorteil, zu Beginn des Reaktionsverlaufs die Zugabe von 2-Methoxyethanol zeitlich und räumlich derart zu wählen, dass der Quotient CR/CA möglichst im oberen Bereich des erfindungsgemäßen Bereichs liegt, also auch tatsächlich bis zu 0,80 beträgt. Mit fortlaufendem Reaktionsverlauf nimmt der Quotient CR/CA natürlich ab und würde bei einem vollständigen Umsatz an 2-Methoxyethanol rechnerisch 0 betragen. Um eine allzu lange Reaktionszeit zu vermeiden, bricht man die Reaktion jedoch, wie einige Absätze weiter unten beschrieben, bei Erreichen eines gewissen Umsatzes an 2-Methoxyethanol ab. Der Quotient CR/CA liegt dabei dann üblicherweise im Bereich von 0 bis 0,1, bevorzugt von 0 bis 0,05 und besonders bevorzugt von 0 bis 0,02.

Im Unterschied zur Lehre der vorliegenden Erfindung wurde beispielsweise in Beispiel 1 der DE 29 36 123 A das Verfahren diskontinuierlich ausgeführt und die Gesamtmenge an 2-Methoxyethanol vor der Zugabe von Sauerstoff im Reaktor vorgelegt. Der Quotient CR/CA betrug somit zu Beginn der Umsetzung 1. Auch in den Beispielen 1 bis 5 der CN 104892390 A zur diskontinuierlichen Reaktionsführung betrug der Quotient CR/CA zu Beginn der Umsetzung ebenfalls 1.

In Beispiel 4 der DE 29 36 123 A wurde das Verfahren kontinuierlich durchgeführt und eine wässrige 2-Methoxyethanol-Lösung zusammen mit Sauerstoff durch ein Edelstahlrohr geleitet. Der Quotient CR/CA betrug somit eingangsseitig ebenfalls 1.

Das erfindungsgemäße Verfahren hebt sich somit deutlich vom Stand der Technik ab.

Das beim erfindungsgemäßen Verfahren zuzugebende 2-Methoxyethanol kann als Reinstoff oder im Gemisch mit weiteren Komponenten der Reaktionsvorrichtung zugeführt werden. Bei der Auswahl der weiteren Komponenten sollten vorteilhafterweise solche Komponenten vermieden werden, die einen negativen Einfluss auf das Reaktionsgeschehen ausüben und/oder nur aufwendig vom Reaktionsgemisch zu trennen sind. Um eine möglichst einfache Aufarbeitung zu gewährleisten, ist es vorteilhaft, 2-Methoxyethanol in möglichst hoher Reinheit von $\geq$ 99 Gew.-%, besonders bevorzugt $\geq$ 99,5 Gew.-% und ganz besonders bevorzugt $\geq$ 99,8 Gew.-% einzusetzen.

Auch wenn Wasser bei der erfindungsgemäßen Oxidation von 2-Methoxyethanol in stöchiometrischer Menge gebildet wird, wird bereits zu Beginn der Reaktion Wasser als Lösungsmittel zugeführt, um 2-Methoxyethanol in verdünnter Lösung zur Verfügung zu stellen. Dies kann beispielsweise derart erfolgen, dass bereits zu Beginn der Reaktion Wasser oder ein Gemisch aus Wasser und 2-Methoxyethanol vorgelegt wird. Dies ist beispielsweise beim halbkontinuierlichen Verfahren üblicherweise der Fall. Beim kontinuierlichen Verfahren werden üblicherweise 2-Methoxyethanol und Wasser kontinuierlich zugeführt. Je nach Reaktionsführung können 2-Methoxyethanol und Wasser getrennt oder zusammen als wässrige 2-Methoxyethanol-Lösung zugeführt werden.

Das Gewichtsverhältnis von Wasser zu 2-Methoxyethanol beträgt beim halbkontinuierlichen Verfahren in Bezug auf die insgesamt eingesetzten Massen an Wasser und 2-Methoxyethanol sowie beim kontinuierlichen Verfahren in Bezug auf die der Reaktionsvorrichtung zugeführten Massenströme an Wasser und 2-Methoxyethanol, jeweils üblicherweise ≥ 1,5, bevorzugt ≥ 2, sowie üblicherweise ≤ 10, bevorzugt ≤ 5 und besonders bevorzugt ≤ 3.

Grundsätzlich ist es möglich, das erfindungsgemäße Verfahren in Gegenwart eines weiteren Lösungsmittels durchzuführen. Dieses sollte unter den vorliegenden Reaktionsbedingungen inert sein und sich anschließend leicht abtrennen lassen. Im Hinblick auf eine einfache Reaktionsführung und Aufarbeitung sowie den Ziel, 2-Methoxyethanol in möglichst hoher Reinheit zu erhalten, ist es jedoch von Vorteil, kein weiteres Lösungsmittel zuzugeben.

Sauerstoff wird entweder in reiner Form oder verdünnt mit anderen Gasen, beispielsweise in Form von Luft oder einem $O_2/N_2$-Gemisch zugegeben. Um bei einem vorgegebenen Sauerstoff-Partialdruck das Gasvolumen so klein wie möglich zu halten, ist der Einsatz eines Gases mit möglichst hohem Sauerstoff-Gehalt vorteilhaft. Bevorzugt setzt man daher ein Sauerstoff-haltiges Gas mit einem Gehalt von ≥ 90 Vol.-%, besonders bevorzugt von ≥95 Vol.-%, ganz besonders bevorzugt von ≥99 Vol.-% und insbesondere von ≥ 99,5 Vol.-% ein.

Beim erfindungsgemäßen Verfahren beträgt der Sauerstoff-Partialdruck 0,01 bis 2 MPa, bevorzugt ≥ 0,02 MPa und besonders bevorzugt ≥ 0,1 MPa, sowie bevorzugt ≤ 1 MPa und besonders bevorzugt ≤ 0,3 MPa.

Die erfindungsgemäße Oxidation von 2-Methoxyethanol erfolgt bei einer Temperatur von 20 bis 100°C, bevorzugt ≥ 30°C und besonders bevorzugt ≥ 40°C, sowie bevorzugt ≤ 80°C und besonders bevorzugt ≤ 60°C.

Der beim erfindungsgemäßen Verfahren einzusetzende heterogene Katalysator enthält Platin als katalytische aktive Komponente. Üblicherweise ist das Platin auf einem Träger fixiert.

Als Träger können die verschiedensten Materialien eingesetzt werden. Als Beispiele seien anorganische Oxide wie etwa Aluminiumoxid, Zirkoniumoxid, Titandioxid, Siliziumoxid, anorganische Silikate wie etwa Aluminiumsilikat, Kohle oder Polymere genannt. Es können natürlich auch Mischungen verschiedener Trägermaterialien eingesetzt werden. Bevorzugt ist der Einsatz von Kohle als Träger.

Der Katalysator enthält im Allgemeinen 0,1 bis 10 Gew.-%, besonders bevorzugt ≥ 0,5 Gew.-% und ganz besonders bevorzugt ≥ 1 Gew.-%, sowie besonders bevorzugt ≤ 8 Gew.-% und ganz besonders bevorzugt ≤ 6 Gew.-% Platin, jeweils bezogen auf die Gesamtmasse des heterogenen Katalysators.

Besonders bevorzugt setzt man beim erfindungsgemäßen Verfahren einen heterogenen Katalysator enthaltend 0,1 bis 10 Gew.-% Platin auf Kohle ein.

Der heterogene, geträgerte Katalysator kann in verschiedenen geometrischen Formen und Größen, wie etwa als Pulver oder Formkörper, eingesetzt werden. Pulverförmige Katalysatoren können beispielsweise in Suspensionsfahrweise betrieben werden. Bei einer Festbettfahrweise setzt man bevorzugt Formkörper ein, wie beispielsweise Granulat, Zylinder, Hohlzylinder, Kugeln oder Extrudate. Die Formkörper sind dann üblicherweise nach den bekannten Methoden im Reaktor fixiert. Im Falle von Katalysatorformkörpern weisen diese bevorzugt eine mittlere Partikelgröße von 1 bis 10 mm auf.

Bevorzugt setzt man jedoch den Katalysator in Form eines Pulvers ein. Der pulverförmige Katalysator liegt dann im Reaktor in Suspension vor. Um einen Austrag aus dem Reaktionssystem zu verhindern, wird hierbei üblicherweise ein Filter zum Rückhalt des Suspensionskatalysators eingesetzt. Als Beispiel eines üblicherweise geeigneten Filters sei der Querstormfilter genannt.

Unabhängig von der geometrischen Form und Größe der Katalysatorpartikel liegt das Platin im Allgemeinen in Form von Partikeln mit einem mittleren Durchmesser von 0,1 bis 50 nm, gemessen über Röntgenbeugung, vor. Es können jedoch auch kleinere oder größere Partikel vorliegen.

Bei der Herstellung des heterogenen, geträgerten Katalysators wird das Platin im Allgemeinen durch geeignete Methoden auf den Träger aufgebracht.

Platin wird dabei üblicherweise aus Lösungen geeigneter Salze auf den Träger aufgebracht. Geeignete Platinsalze sind beispielsweise solche, welche in wässrigen beziehungsweise wässrig-sauren Medien löslich sind und aus denen durch eine Erhöhung des pH-Wertes eine Platinverbindung ausgefällt werden kann. Als bevorzugte Beispiele eines geeigneten Platinsalzes seien Platin(II)nitrat, Platin(IV)chlorid und Hexachloroplatinsäure Hexahydrat genannt.

Als pH-Wert erhöhende Mittel kommen insbesondere wässrige Lösungen alkalischer Salze in Frage, wie beispielsweise Alkalicarbonate, bevorzugt Natriumcarbonat.

Zur Aufbringung der unlöslichen oder schwer löslichen Platinverbindungen sind prinzipiell die verschiedensten Methoden möglich. In einer bevorzugten Ausführungsform wird der Träger in einer geeigneten Apparatur, beispielsweise einer rotierenden Trommel oder einem gerührten Behälter, in überstehender Flüssigkeit, beispielsweise Wasser, vorgelegt und mit der Lösung des Platinsalzes sowie der pH-Wert erhöhenden Lösung versetzt. Dabei ist es möglich, zuerst das Platinsalz und anschließend die pH-Wert erhöhende Lösung, oder zuerst die pH-Wert erhöhende Lösung und anschließend das Platinsalz, oder beide alternierend oder auch gleichzeitig zuzufügen.

Bevorzugt wird der Träger in Wasser vorgelegt und der pH-Wert mit der pH-Wert erhöhenden Lösung auf einen Wert eingestellt, bei dem das Platinsalz als unlösliche oder schwer lösliche Platinverbindung ausfällt. Anschließend wird unter Durchmischung die Lösung des Platinsalzes zugefügt, wobei der pH-Wert durch weitere Zugabe der den pH-Wert erhöhenden Lösung in einem Bereich gehalten wird, in dem das Platinsalz als unlösliche oder schwer lösliche Platinverbindung ausfällt. Das Gewichtsverhältnis zwischen der insgesamt zuzugebender Flüssigkeitsmenge und dem Träger liegt im Allgemeinen bei einem Wert von 1 bis 100.

Nach erfolgter Auffällung wird der, die Platinverbindung enthaltende Träger isoliert, getrocknet und mit Wasserstoff zur Reduktion des Platins behandelt. Die Reduktion kann dabei sowohl mit reinem Wasserstoff als auch mit einem durch Inertgas verdünnten Wasserstoff durchgeführt werden. Geeignete Inertgase sind beispielsweise Stickstoff oder Edelgase.

Bei der Imprägnierung wird die Lösung eines geeigneten Platinsalzes in einer geeigneten Apparatur, beispielsweise einer rotierenden Mischtrommel auf den Träger ausgesprüht. Die insgesamt aufzusprühende Menge an Platinsalz-Lösung liegt dabei bevorzugt bei oder unterhalb der Flüssigkeitsaufnahme des vorgelegten Trägers. Bei der Imprägnierung werden bevorzugt Platinsalze eingesetzt, welche sich durch Tempern rückstandsfrei in elementares Platin umwandeln. Bevorzugte Platinsalze zur Imprägnierung sind beispielsweise Platin(II)nitrat sowie Hexachloroplatinsäure.

Der heterogene, geträgerte Katalysator weist im Allgemeinen eine BET-Oberfläche von $\geq 1\ m^2/g$ und $\leq 10000\ m^2/g$, bestimmt nach DIN ISO 9277:2014-01, auf. Beim Einsatz von Kohle als Träger liegt die BET-Oberfläche bevorzugt im Bereich von $\geq 500\ m^2/g$ und $\leq 10000\ m^2/g$.

Beim erfindungsgemäßen Verfahren ist es grundsätzlich möglich, in ein und derselben Reaktionsvorrichtung auch verschiedene Katalysatoren entlang des räumlichen Reaktionsverlaufs einzusetzen. So ist es beispielsweise sogar möglich, in kaskadierten oder kompartimierten Reaktoren einen Teil mit einem Festbettkatalysator und einen anderen Teil mit einem Suspensionskatalysator zu betreiben.

Hinsichtlich der Menge des einzusetzenden Katalysators ist das erfindungsgemäße Verfahren sehr flexibel. Grundsätzlich gilt, dass ein höherer Gehalt an Platin zu einer höheren katalytischen Aktivität führt und somit im Regelfall auch mehr 2-Methoxyethanol pro Zeiteinheit umgesetzt werden kann. Beim halbkontinuierlichen Verfahren beträgt das molare Verhältnis der insgesamt eingesetzten Menge an 2-Methoxyethanol zu der in der Reaktionsvorrichtung vorhandenen Menge an Platin 1 bis 10000, bevorzugt $\geq 10$ und besonders bevorzugt $\geq 100$, sowie bevorzugt $\leq 5000$ und besonders bevorzugt $\leq 1000$. Beim kontinuierlichen Verfahren beträgt das molare Verhältnis der pro Zeiteinheit der Reaktionsvorrichtung zugeführten Menge an 2-Methoxyethanol zu der in der Reaktionsvorrichtung vorhandenen Menge an Platin 1 bis 500 pro Stunde, bevorzugt $\geq 5$ pro Stunde und besonders bevorzugt $\geq 20$ pro Stunde, sowie bevorzugt $\leq 300$ pro Stunde und besonders bevorzugt $\leq 200$ pro Stunde.

Beim halbkontinuierlichen Verfahren führt man der Reaktionsvorrichtung 2-Methoxyethanol üblicherweise über einen Zeitraum von 1 bis 10 Stunden, bevorzugt $\geq 2$ Stunden, sowie bevorzugt $\leq 6$ Stunden zu. Nach Bedarf sind jedoch auch deutliche Abweichungen zu den genannten Zeitwerten möglich. Es sei auch explizit erwähnt, dass die Zugabemenge an 2-Methoxyethanol auch unregelmäßig, beispielsweise intermittierend, ansteigend, abfallend oder schwankend, erfolgen kann. Auf die obigen Ausführungen zum zeitlichen und räumlichen Verlauf des Quotienten CR/CA über den Reaktionsverlauf hinweg sei hingewiesen. Nach Beendigung der Zugabe von 2-Methoxyethanol wird das Reaktionsgemisch im Allgemeinen noch zur Nachreaktion von bisher noch nicht umgesetztem 2-Methoxyethanol für einen weiteren Zeitraum bei Reaktionsbedingungen belassen und erst anschließend weiter aufgearbeitet. Die gesamte Reaktionszeit ab Reaktionsbeginn beträgt beim halbkontinuierlichen Verfahren üblicherweise 2 bis 20 Stunden, bevorzugt $\geq 9$ Stunden, sowie bevorzugt $\leq 14$ Stunden.

Beim kontinuierlichen Verfahren führt man 2-Methoxyethanol und Wasser der Reaktionsvorrichtung derart zu, dass der Massenstrom an 2-Methoxyethanol und Wasser bezogen auf das gesamte Reaktorvolumen in der Reaktionsvorrichtung üblicherweise 0,05 bis 0,5 pro Stunde, bevorzugt $\leq 0{,}11$ pro Stunde, sowie bevorzugt $\geq 0{,}07$ pro Stunde beträgt.

Die Reaktionszeit richtet sich hauptsächlich nach dem gewünschten Umsatz. Je länger die Reaktionszeit unter sonst vergleichbaren Bedingungen, desto höher ist im Regelfall auch der Umsatz an 2-Methoxyethanol. Da nicht umgesetztes 2-Methoxyethanol jedoch vergleichsweise leicht abgetrennt und wieder rückgeführt oder in einem nachfolgendem Ansatz erneut eingesetzt werden kann, ist es im Sinne einer effizienten Verfahrensführung durchaus vorteilhaft, keinen Vollumsatz anzustreben, sondern bewusst auf Teilumsatz zu fahren. Dadurch wird pro Zeiteinheit und Reaktorvolumen ein höherer Umsatz an 2-Methoxyethanol erreicht. Aus diesem Grund ist es vorteilhaft, nur einen Umsatz an 2-Methoxyethanol von 80 bis 99%, bevorzugt $\geq 90\%$ und besonders bevorzugt $\geq 93\%$, sowie bevorzugt $\leq 98\%$ und besonders bevorzugt $\leq 97\%$ anzustreben.

Als Reaktoren in der Reaktionsvorrichtung kommen beim erfindungsgemäßen Verfahren prinzipiell Reaktionsapparate in Frage, welche für die Durchführung exothermer, heterogenkatalysierter Gas-Flüssig-Reaktionen geeignet sind und halbkontinuierlich oder kontinuierlich betrieben werden können. Als Beispiele seien genannt Rührkessel, Rieselbettreaktor, Blasensäulenreaktor, Strahlschlaufenreaktor sowie Kaskaden der genannten Reaktoren. Beim halbkontinuierlichen Verfahren sind Rührkessel, Rieselbettreaktor und Blasensäulenreaktor sowie beim kontinuierlichen Verfahren Rührkesselkaskade, Rieselbettreaktor-Kaskade, kaskadierter Blasensäulenreaktor und kaskadierter Strahlschlaufenreaktor bevorzugt. Die beim kontinuierlichen Verfahren üblicherweise eingesetzten Reaktorkaskaden enthalten im Allgemeinen 2 bis 10, bevorzugt 2 bis 6 und besonders bevorzugt 2 bis 4 hintereinandergeschaltete Reaktoren. Im Falle eines kaskadierten Blasensäulenreaktors oder eines kaskadierten Strahlschlaufenreaktors enthalten diese im Allgemeinen 2 bis 8, bevorzugt 2 bis 5 und besonders bevorzugt 2 bis 3 hintereinander angeordnete Kompartimente.

Wie eingangs bereits erwähnt, bildet der Reaktor beziehungsweise die Verschaltung mehrerer Reaktoren zusammen mit den Wärmetauschervorrichtungen und etwaiger Vorrichtungen zur partiellen Rückführung des Reaktionsgemisches die sogenannte Reaktionsvorrichtung.

Aufgrund der hohen Korrosivität wässriger 2-Methoxyessigsäure ist es empfehlenswert, die Reaktionsvorrichtung oder zumindest jene Teile, die im direkten Kontakt zur wässrigen 2-Methoxyessigsäure stehe, aus einem korrosionsfesten Werkstoff anzufertigen. Geeignete Werkstoffe sind beispielsweise hochlegierte Edelstähle, Nickelbasis Legierungen, Titan oder Titan-Palladium Legierung, Zirkon oder Tantal. Bevorzugt ist der Einsatz hochlegierter Edelstähle. Alternativ ist gegebenenfalls auch eine Auskleidung der betroffenen Apparateteile möglich. Denkbar sind beispielsweise säureresistente Kunststoffe oder Emaille.

Die Reaktionsvorrichtung ist üblicherweise mit einer Wärmetauschervorrichtung ausgestattet. Diese dient vor Beginn der Reaktion zur Aufheizung sowie während der Reaktion zur Abführung der produzierten Wärme und somit zur Aufrechterhaltung der gewünschten Reaktionstemperatur. Je nach Ausgestaltung kann die Wärmetauschervorrichtung innerhalb oder außerhalb des Reaktors liegen. Liegt diese innerhalb, so handelt es sich üblicherweise um Wärmetauscherschlangen oder Wärmetauscherplatten. Besonders vorteilhaft ist der Einsatz einer außerhalb des Reaktors befindlichen Wärmetauschervorrichtung. Hierbei wird dann im Betrieb ein Flüssigkeitsstrom aus dem Reaktor kontinuierlich entnommen, zur außenliegenden Wärmetauschervorrichtung geleitet und nachfolgend wieder in den Reaktor zurückgeführt. Unabhängig davon, ob es sich um eine innenliegende oder außenliegende Wärmetauschervorrichtung handelt, ist diese vorteilhafterweise so dimensioniert, dass diese auch die während des Reaktionsverlaufs maximal produzierter Wärme abzuführen vermag und somit die gewünschte Reaktionstemperatur nicht überschritten wird.

Zur Druckhaltung sowie zur gezielten Ausschleusung von Abgas, wie beispielsweise Kohlendioxid, welches sich gegebenenfalls durch Überoxidation bilden kann, oder über die Zufuhr des sauerstoffhaltigen Gases eingeführte Inertgase, enthält die Reaktionsvorrichtung bevorzugt ein Druckhalteventil. Über dieses wird der Reaktionsdruck auf den gewünschten Maximalwert begrenzt und bei Erreichen des Maximalwertes Abgas aus der Reaktionsvorrichtung abgeführt.

Das beim erfindungsgemäßen Verfahren erhaltene Reaktionsgemisch enthält neben der gebildeten 2-Methoxyessigsäure natürlich Wasser, nicht ungesetzten 2-Methoxyethanol, geringe Mengen an Methoxyessigsäure-2-methoxyethylester sowie geringe Mengen weiterer Nebenprodukte. Dieses Reaktionsgemisch wird zum Erhalt möglichst reiner 2-Methoxyessigsäure anschließend aufgearbeitet. Hierbei zeigt sich der große Vorteil der vorliegenden Erfindung. Da durch die erfindungsgemäße Zugabe von 2-Methoxyethanol zur Reaktionsvorrichtung 2-Methoxyessigsäure mit einem deutlich geringeren Gehalt an unerwünschtem Methoxyessigsäure-2-methoxyethylester gebildet wird, ist es in vielen Fällen ausreichend, aus dem erhaltenen Reaktionsgemisch nur die Leichtsieder Wasser und 2-Methoxyethanol durch Verdampfung zu entfernen. Die dadurch erhaltene 2-Methoxyessigsäure enthält erfindungsgemäß nur geringe Mengen an Methoxyessigsäure-2-methoxyethylester und kann oftmals direkt ohne weitere Aufreinigung als Synthesebaustein in Folgereaktionen eingesetzt werden.

Die Verdampfung der Leichtsieder Wasser und 2-Methoxyethanol kann beispielsweise in einem Sambay-Verdampfer oder einer Destillationskolonne erfolgen. Je nach Bauart des verwendeten Reaktors ist es gegebenenfalls möglich, die Verdampfung der Leichtsieder Wasser und 2-Methoxyethanol nach Beendigung der Reaktion sogar direkt im Reaktor durchzuführen.

Um die Verdampfung der Leichtsieder Wasser und 2-Methoxyethanol mit möglichst wenig Energieeintrag durchzuführen, führt man diese bevorzugt bei Normaldruck oder Unterdruck und besonders bevorzugt bei Unterdruck durch.

Falls dennoch eine noch reinere 2-Methoxyessigsäure erhalten werden soll, kann das, nach der Verdampfung der Leichtsieder Wasser und 2-Methoxyethanol erhaltene Produkt weiter aufgereinigt werden. Da eine destillative Trennung von 2-Methoxyessigsäure und Methoxyessigsäure-2-methoxyethylester aufgrund deren sehr ähnlicher Siedepunkte relativ aufwendig ist, kommen in erster Linie alternative Verfahren in Frage. Eine alternative Möglichkeit ist beispielsweise die in DE 33 45 807 A beschriebene Kristallisation.

Durch einfaches Verdampfen der Leichtsieder Wasser und 2-Methoxyethanol kann 2-Methoxyessigsäure bereits in einer Reinheit von $\geq$ 99 Gew.-% erhalten werden. Je nach der Durchführung der 2-Methoxyethanol-Zugabe zur Reaktionsvorrichtung ist auch eine Reinheit von $\geq$ 99,5 Gew.-% in einfacher Art und Weise möglich. Je niedriger der Quotient CR/CA ist, desto weniger Methoxyessigsäure-2-methoxyethylester werden in der Regel gebildet und desto reiner ist die 2-Methoxyessigsäure.

Im Folgenden sind einige mögliche Ausführungsformen des erfindungsgemäßen Verfahrens erläutert. In den Abbildungen Fig. 1 bis 7 werden folgende Abkürzungen benutzt:

A 2-Methoxyethanol (gegebenenfalls als wässrige Lösung)
B Sauerstoff
C Wasser
M Reaktionsgemisch
Z Abgas (druckgeregelt)

Die halbkontinuierliche Verfahrensführung erfolgt bevorzugt in einem Rührkessel, einem Rieselbettreaktor oder einem Blasensäulenreaktor.

Fig. 1a und 1b zeigen stark vereinfacht mögliche Reaktionsvorrichtungen zur Durchführung des halbkontinuierlichen Verfahrens unter Einsatz eines Rührkessels. In Fig. 1a befindet sich die Wärmetauschervorrichtung innerhalb, und in Fig. 1b außerhalb des Rührkessels. Der Rührkessel kann sowohl in Suspensionsfahrweise als auch in Festbettfahrweise betrieben werden. Bevorzugt ist der Betrieb in Suspensionsfahrweise. Zu Beginn der Reaktion werden üblicherweise Wasser und der Katalysator im Rührkessel vorgelegt. Zudem ist es möglich, auch einen Teil des einzusetzenden 2-Methoxyethanols bereits zu Beginn vorzulegen. Die Reaktionsvorrichtung wird nun auf Reaktionsbedingungen gebracht, das heißt insbesondere unter Durchmischung auf die gewünschte Temperatur gebracht und durch Aufpressen von Sauerstoff der gewünschte Sauerstoff-Partialdruck eingestellt. 2-Methoxyethanol wird dann zeitlich verteilt bis Erreichen der insgesamt zuzugebenden Masse an 2-Methoxyethanol durch ein Zulaufrohr oder eine Düse zugeführt. Je nach Variante ist es auch möglich, eine wässrige 2-Methoxyethanol-Lösung zuzuführen. Zur Aufrechterhaltung des gewünschten Sauerstoff-Partialdruckes wird während der Reaktion Sauerstoff nachdosiert. Die Zufuhr von Sauerstoff kann ebenfalls beispielsweise durch ein Zulaufrohr oder eine Düse erfolgen. Überschüssiges Gas kann beispielsweise über eine Druckhaltung abgeführt werden. Nach Beendigung der 2-Methoxyethanol-Zugabe ist es in der Regel vorteilhaft, das Reaktionsgemisch für eine gewisse Zeit zur Nachreaktion und somit zur Erhöhung des Umsatzes unter den Reaktionsbedingungen zu belassen. Anschließend wird die Reaktionsvorrichtung in der Regel entspannt, entleert und das Reaktionsgemisch aufgearbeitet.

Fig. 2 zeigt stark vereinfacht eine mögliche Reaktionsvorrichtungen zur Durchführung des halbkontinuierlichen Verfahrens unter Einsatz eines Rieselbettreaktors. Dieser ist vorteilhafterweise mit einem außenliegenden Wärmetauscher ausgestattet. Der Rieselbettreaktor kann sowohl in Suspensionsfahrweise als auch in Festbettfahrweise betrieben werden. Im Falle einer Suspensionsfahrweise dient eine inerte Schüttung oder eine inerte Packung als Rieselbett. Bevorzugt ist der Betrieb in Festbettfahrweise. Zu Beginn der Reaktion werden üblicherweise Wasser und der Katalysator im Rieselbettreaktor vorgelegt. Die Reaktionsvorrichtung wird nun auf Reaktionsbedingungen gebracht, das heißt insbesondere über den außenliegenden Wärmetauscherkreislauf auf die gewünschte Temperatur gebracht und durch Aufpressen von Sauerstoff der gewünschte Sauerstoff-Partialdruck eingestellt. 2-Methoxyethanol wird dann zeitlich verteilt bis Erreichen der insgesamt zuzugebenden Masse an 2-Methoxyethanol der Reaktionsvorrichtung zugeführt. Da die flüssige Phase aufgrund des Wärmetauscherkreislaufs praktisch komplett rückvermischt ist, kann 2-Methoxyethanol grundsätzlich überall in die Reaktionsvorrichtung zudosiert werden. Bevorzugt erfolgt die Zufuhr jedoch im Wärmetauscherkreislauf. Je nach Variante ist es auch möglich, eine wässrige 2-Methoxyethanol-Lösung zuzuführen. Zur Aufrechterhaltung des gewünschten Sauerstoff-Partialdruckes wird während der Reaktion Sauerstoff nachdosiert. Die Zufuhr von Sauerstoff erfolgt bevorzugt unterhalb des Rieselbetts. Alternativ kann Sauerstoff aber auch an anderen Stellen, beispielsweise auch am Reaktorkopf, zugegeben werden. Zur Aufrechterhaltung des gewünschten Sauerstoff-Partialdruckes wird während der Reaktion bei Bedarf Sauerstoff nachdosiert. Überschüssiges Gas kann beispielsweise über eine Druckhaltung abgeführt werden. Während der Reaktionsführung wird das Rieselbett über Düsen, welche sich oberhalb des Rieselbetts befinden, und aus welchen das Reaktionsgemisch aus dem außenliegenden Wärmetauscherkreislauf strömt, berieselt. Beim Betrieb des Rieselbettreaktors befindet sich der Flüssigkeitsspiegel unterhalb des Rieselbetts. Aus dem als Sumpfbereich bezeichneten Bereich wird der Strom für den Wärmetauscherkreislauf entnommen. Nach Beendigung der 2-Methoxyethanol-Zugabe ist es in der Regel vorteilhaft, das Reaktionsgemisch für eine gewisse Zeit zur Nachreaktion und somit zur Erhöhung des Umsatzes unter den Reaktionsbedingungen zu belassen. Anschließend wird die Reaktionsvorrichtung in der Regel entspannt, entleert und das Reaktionsgemisch aufgearbeitet.

Fig. 3 zeigt stark vereinfacht eine mögliche Reaktionsvorrichtungen zur Durchführung des halbkontinuierlichen Verfahrens unter Einsatz eines Blasensäulenreaktors. Dieser ist ebenfalls vorteilhafterweise mit einem außenliegenden Wärmetauscher ausgestattet. Auch der Blasensäulenreaktor kann sowohl in Suspensionsfahrweise als auch in Festbettfahrweise betrieben werden. Im Falle einer Suspensionsfahrweise kann optional eine inerte Schüttung oder eine inerte Packung als Mischelement verwendet werden. Bevorzugt ist der Betrieb in Festbettfahrweise. Zu Beginn der Reaktion werden üblicherweise Wasser und der Katalysator im Blasensäulenreaktor vorgelegt. Die Reaktionsvorrichtung wird nun auf Reaktionsbedingungen gebracht, das heißt insbesondere über den außenliegenden Wärmetauscherkreislauf auf die gewünschte Temperatur gebracht und durch Aufpressen von Sauerstoff der gewünschte Sauerstoff-Partialdruck eingestellt. 2-Methoxyethanol wird dann zeitlich verteilt bis Erreichen der insgesamt zuzugebenden Masse an 2-Methoxyethanol der Reaktionsvorrichtung zugeführt. Da die flüssige Phase aufgrund des Wärmetauscherkreislaufs praktisch komplett rückvermischt ist, kann 2-Methoxyethanol grundsätzlich überall in die Reaktionsvorrichtung zudosiert

werden. Bevorzugt erfolgt die Zufuhr jedoch im Wärmetauscherkreislauf. Je nach Variante ist es auch möglich, eine wässrige 2-Methoxyethanol-Lösung zuzuführen.

Zur Aufrechterhaltung des gewünschten Sauerstoff-Partialdruckes wird während der Reaktion Sauerstoff nachdosiert. Die Zufuhr von Sauerstoff erfolgt über eine oder mehrere unterhalb des Mischelements angebrachte Düsen. Überschüssiges Gas kann beispielsweise über eine Druckhaltung abgeführt werden. Flüssiges Reaktionsgemisch wird üblicherweise aus dem unteren Bereich des Blasensäulenreaktors entnommen und nach Durchströmung des außenliegenden Wärmetauscherkreislaufs oberhalb des Mischelements wieder rückgeführt. Beim Betrieb des Blasensäulenreaktors befindet sich der Flüssigkeitsspiegel oberhalb des Mischelements. Nach Beendigung der 2-Methoxyethanol-Zugabe ist es in der Regel vorteilhaft, das Reaktionsgemisch für eine gewisse Zeit zur Nachreaktion und somit zur Erhöhung des Umsatzes unter den Reaktionsbedingungen zu belassen. Anschließend wird die Reaktionsvorrichtung in der Regel entspannt, entleert und das Reaktionsgemisch aufgearbeitet.

Die kontinuierliche Durchführung erfolgt bevorzugt in einer Rührkesselkaskade, einer Rieselbettreaktor-Kaskade, einem kaskadierten Blasensäulenreaktor oder einem kaskadierten Strahlschlaufenreaktor.

Fig. 4a zeigt stark vereinfacht eine mögliche Reaktionsvorrichtungen zur Durchführung des kontinuierlichen Verfahrens unter Einsatz einer Rührkesselkaskade. Diese besteht aus mehreren hintereinandergeschalteten Rührkesseln. Die Wärmetauschervorrichtung der einzelnen Rührkessel kann sich grundsätzlich innerhalb oder außerhalb des jeweiligen Rührkessels befinden. Bevorzugt befindet sich die jeweilige Wärmetauschervorrichtung in einem außenliegenden Kreislauf. Die Rührkesselkaskade kann sowohl in Suspensionsfahrweise als auch in Festbettfahrweise betrieben werden. Bevorzugt ist der Betrieb in Suspensionsfahrweise. In diesem Fall wird der Austrag eines jeden, Suspensionskatalysator enthaltenden Rührkessels bevorzugt über einen Querstromfilter entnommen, um den Suspensionskatalysator im jeweiligen Rührkessel zurückzuhalten. Zu Beginn der Reaktion werden üblicherweise Wasser und der Katalysator im Rührkessel vorgelegt. Zudem ist es möglich, auch bereits etwas 2-Methoxyethanol zu Beginn vorzulegen, beispielsweise im ersten Rührkessel. Die Reaktionsvorrichtung wird nun auf Reaktionsbedingungen gebracht, das heißt insbesondere unter Durchmischung auf die gewünschte Temperatur gebracht und durch Aufpressen von Sauerstoff der gewünschte Sauerstoff-Partialdruck eingestellt. 2-Methoxyethanol, Sauerstoff und Wasser werden nun kontinuierlich der Reaktionsvorrichtung zugeführt. Sauerstoff wird bevorzugt jedem einzelnen Rührkessel zugeführt. Überschüssiges Gas kann beispielsweise über eine Druckhaltung abgeführt werden. Die Zufuhr von 2-Methoxyethanol erfolgt räumlich verteilt im ersten bis zum vorletzten Rührkessel. Der letzte Rührkessel dient üblicherweise als Nachreaktionszone und weist daher im Allgemeinen keine Zugabe von 2-Methoxyethanol auf. Wasser wird bevorzugt nur im ersten Rührkessel zugeführt. Das Reaktionsgemisch wird jeweils kontinuierlich einem Rührkessel entnommen und dem nachfolgenden Rührkessel zugeführt. Das Reaktionsgemisch wird kontinuierlich aus dem letzten Rührkessel entnommen, in der Regel entspannt und aufgearbeitet.

Eine etwas abgewandelte Ausführungsform einer Rührkesselkaskade ist ein kaskadierter Rührkessel. Fig. 4b zeigt eine stark vereinfachte Reaktionsvorrichtung zur Durchführung des kontinuierlichen Verfahrens unter Einsatz eines kaskadierten Rührkessels. Dieser umfasst in einem Reaktionsbehälter mehrere hintereinandergeschaltete Rührkessel-Kompartimente, die jeweils mit einem Überlauf in das nächste Kompartiment versehen sind. Die Wärmetauschervorrichtung der einzelnen Kompartimente kann sich grundsätzlich innerhalb oder außerhalb des jeweiligen Kompartiments befinden. Bevorzugt befindet sich die jeweilige Wärmetauschervorrichtung in einem außenliegenden Kreislauf. Der kaskadierte Rührkessel kann sowohl in Suspensionsfahrweise als auch in Festbettfahrweise betrieben werden. Bevorzugt ist der Betrieb in Suspensionsfahrweise. In diesem Fall wird der Austrag eines jeden, Suspensionskatalysator enthaltenden Kompartiments bevorzugt über einen Querstromfilter entnommen, um den Suspensionskatalysator im jeweiligen Kompartiment zurückzuhalten. Die Zugabe von 2-Methoxyethanol und Wasser erfolgt im Prinzip wie bei der Rührkesselkaskade. Das letzte Kompartiment dient der Nachreaktion und weist daher üblicherweise keine Zugabe von 2-Methoxyethanol auf. Im Gegensatz zur Rührkesselkaskade wird beim kaskadierten Rührkessel der Sauerstoff jedoch üblicherweise an zentraler Stelle zudosiert. Daher ist im Allgemeinen auch eine zentrale Druckhaltung ausreichend. Das Reaktionsgemisch wird kontinuierlich aus dem letzten Kompartiment entnommen, in der Regel entspannt und aufgearbeitet.

Fig. 5 zeigt stark vereinfacht eine mögliche Reaktionsvorrichtungen zur Durchführung des kontinuierlichen Verfahrens unter Einsatz einer Rieselbettreaktor-Kaskade. Diese besteht aus mehreren hintereinandergeschalteten Rieselbettreaktoren mit bevorzugt außenliegendem Wärmetauscherkreislauf. Der grundsätzliche Aufbau und Betrieb eines Rieselbettreaktors ist bereits bei der halbkontinuierlichen Fahrweise beschrieben. Auch die Rieselbettreaktor-Kaskade kann sowohl in Suspensionsfahrweise als auch in Festbettfahrweise betrieben werden. Bevorzugt ist der Betrieb in Festbettfahrweise. 2-Methoxyethanol, Sauerstoff und Wasser werden kontinuierlich der Reaktionsvorrichtung zugeführt. Sauerstoff wird bevorzugt jedem einzelnen Rieselbettreaktor zugeführt. Überschüssiges Gas kann beispielsweise über eine Druckhaltung abgeführt werden. Die Zufuhr von 2-Methoxyethanol erfolgt räumlich verteilt im ersten bis zum vorletzten Rieselbettreaktor. Der letzte Rieselbettreaktor dient üblicherweise als Nachreaktionszone und weist daher im Allgemeinen keine Zugabe von 2-Methoxyethanol auf. Wasser wird bevorzugt nur im ersten Rieselbettreaktor zugeführt. Das Reaktionsgemisch wird jeweils kontinuierlich einem Rieselbettreaktor entnommen und dem nachfolgenden Rieselbettreaktor zugeführt. Das Reaktionsgemisch wird kontinuierlich aus dem letzten Rieselbettreaktor entnommen, in der Regel entspannt und aufgearbeitet.

Fig. 6 zeigt stark vereinfacht eine mögliche Reaktionsvorrichtungen zur Durchführung des kontinuierlichen Verfahrens unter Einsatz eines kaskadierten Blasensäulenreaktors. Dieser ist ähnlich eines Blasensäulenreaktors aufgebaut, umfasst aber mehrere hintereinandergeschaltete Blasensäulen-Kompartimente. Diese sind üblicherweise durch geeignete Trennvorrichtungen, beispielsweise Lochblechen, voneinander abgetrennt. Obwohl diese für den von unten aufsteigenden Sauerstoff als auch für das flüssige Reaktionsgemisch durchlässig sind, stellen sie dennoch einen Strömungswiderstand dar und reduzieren die Rückvermischung. In den einzelnen Kompartimenten befindet sich jeweils heterogener Katalysator. Der kaskadierte Blasensäulenreaktor kann sowohl in Suspensionsfahrweise als auch in Festbettfahrweise betrieben werden. Im Falle einer Suspensionsfahrweise dient jeweils eine inerte Schüttung oder eine inerte Packung als Mischelement. Bevorzugt ist der Betrieb in Festbettfahrweise. Mit Ausnahme des obersten Kompartiments wird jeweils oberhalb des jeweiligen Mischelements Reaktionsgemisch entnommen, über einen Wärmetauscherkreislauf geführt und unterhalb des jeweiligen Mischelements wieder rückgeführt. Das oberste Kompartiment dient als Nachreaktionszone und bedarf keiner Kühlung. 2-Methoxyethanol, Sauerstoff und Wasser werden kontinuierlich der Reaktionsvorrichtung zugeführt. Sauerstoff wird über eine oder mehrere Düsen unterhalb des ersten Mischelements zugeführt. Die Zufuhr von 2-Methoxyethanol erfolgt räumlich verteilt über die jeweiligen Wärmetauscherkreisläufe des ersten bis vorletzten Kompartiments. Da die flüssige Phase aufgrund des Wärmetauscherkreislaufs in jedem Kompartiment praktisch komplett rückvermischt ist, kann 2-Methoxyethanol grundsätzlich innerhalb des jeweiligen Kompartiments überall zudosiert werden. Wasser wird bevorzugt nur unterhalb des ersten Kompartiments zugeführt. Beim Betrieb des kaskadierten Blasensäulenreaktors befindet sich der Flüssigkeitsspiegel oberhalb des obersten Mischelements. Überschüssiges Gas kann beispielsweise über eine Druckhaltung abgeführt werden. Das Reaktionsgemisch wird kontinuierlich oberhalb des letzten Mischelements entnommen, in der Regel entspannt und aufgearbeitet.

Fig. 7 zeigt stark vereinfacht eine mögliche Reaktionsvorrichtungen zur Durchführung des kontinuierlichen Verfahrens unter Einsatz eines kaskadierten Strahlschlaufenreaktors. Dieser ist ähnlich eines Strahlschlaufenreaktors aufgebaut, umfasst aber mehrere hintereinandergeschaltete Strahlschlaufen-Kompartimente, wobei das oberste Kompartiment ohne Strahlschlaufen-Einbauten ausgestattet ist und als Nachreaktionszone dient. Die einzelnen Kompartimente sind üblicherweise durch geeignete Trennvorrichtungen, beispielsweise Lochblechen, voneinander abgetrennt. Diese stellen einen Strömungswiderstand dar und reduzieren die Rückvermischung. Wie für Strahlschlaufenreaktoren üblich umfassen die Strahlschlaufen-Einbauten jeweils eine Strahldüse, ein Impulsrohr und eine Prallplatte. Beim Betrieb des kaskadierten Strahlschlaufenreaktors wird Reaktionsgemisch jeweils unterhalb der Prallplatte entnommen, über den Wärmetauscherkreislauf geführt und zusammen mit frisch zugeführtem Sauerstoff über die im Impulsrohr befindliche Strahldüse rückgeführt und somit eine intensive Vermischung innerhalb des jeweiligen Strahlschlaufen-Kompartiments sichergestellt. In den einzelnen Kompartimenten befindet sich jeweils heterogener Katalysator. Der kaskadierte Strahlschlaufenreaktor kann sowohl in Suspensionsfahrweise als auch in Festbettfahrweise betrieben werden. Bevorzugt ist der Betrieb in Festbettfahrweise. Im Falle einer Festbettfahrweise befindet sich der Katalysator fixiert in dem ringförmigen Bereich zwischen Impulsrohr und Reaktorwandung. In der Nachreaktionszone liegt im einfachsten Fall der Katalysator in Form eines klassischen Festbetts ohne umgebende Strahlschlaufen-Einbauten vor (wie in der Abbildung dargestellt). Alternativ können jedoch auch in der Nachreaktionszone Strahlschlaufen-Einbauten samt externer Kreislaufpumpe zugegen sein und der Katalysator fixiert in dem ringförmigen Bereich zwischen Impulsrohr und Reaktorwandung vorliegen. Im Falle einer Suspensionsfahrweise ist auf jeden Fall sicherzustellen, dass auch die Nachreaktionszone aktiv durchmischt wird. Dies kann beispielsweise über einen mechanischen Rührer oder Strahlschlaufen-Einbauten mit externer Kreislaufpumpe erreicht werden. Unabhängig davon, ob der kaskadierte Strahlschlaufenreaktor in Suspensions- oder Festbettfahrweise betrieben wird, wird 2-Methoxyethanol räumlich verteilt mit Ausnahme der Nachreaktionszone den jeweiligen Strahlschlaufen-Kompartimenten zugeführt. Da die flüssige Phase aufgrund des Wärmetauscherkreislaufs und der intensiven Vermischung in jedem Strahlschlaufen-Kompartiment praktisch komplett rückvermischt ist, kann 2-Methoxyethanol grundsätzlich innerhalb des jeweiligen Kompartiments überall zudosiert werden. Wasser wird bevorzugt nur unterhalb des ersten Kompartiments zugeführt. Beim Betrieb des kaskadierten Strahlschlaufenreaktors befindet sich der Flüssigkeitsspiegel oberhalb des obersten Kompartiments. Überschüssiges Gas kann beispielsweise über eine Druckhaltung abgeführt werden. Das Reaktionsgemisch wird kontinuierlich aus dem oberen Bereich des obersten Kompartiments, jedoch unterhalb des Flüssigkeitsspiegels entnommen, in der Regel entspannt und aufgearbeitet.

Das erfindungsgemäße Verfahren ermöglicht die Herstellung von 2-Methoxyessigsäure in hoher Selektivität, Ausbeute und Reinheit. Das Verfahren ist einfach durchzuführen und basiert auf den leicht zugänglichen Einsatzstoff 2-Methoxyethanol. Die erfindungsgemäß erhältliche 2-Methoxyessigsäure wird in deutlich höherer Reinheit erhalten als eine nach dem Stand der Technik hergestellte 2-Methoxyessigsäure. Insbesondere enthält die erfindungsgemäß erhältliche 2-Methoxyessigsäure deutlich geringere Mengen am unerwünschten Nebenprodukt Methoxyessigsäure-2-methoxyethylester. Des Weiteren ermöglicht das erfindungsgemäße Verfahren trotz einer höheren Selektivität zu 2-Methoxyessigsäure und einer geringeren Bildung von Methoxyessigsäure-2-methoxyethylester eine hinsichtlich 2-Methoxyethanol und 2-Methoxyessigsäure konzentriertere Fahrweise und somit den Einsatz eines kleineres Reaktorvolumen. Die geringere Bildung von Nebenprodukten, insbesondere von Methoxyessigsäure-2-methoxyethylester, sowie die höher konzentrierte Fahrweise ermöglichen eine apparativ einfachere und energiesparendere Aufarbeitung. Zudem weist die erhaltene 2-Methoxyessigsäure eine niedrige Farbzahl auf.

Beispiele

Beispiele 1 bis 5

In einem 1,6 L-Reaktionskalorimeter mit Hohlwellen-Begasungsrührer wurden jeweils 375 g Wasser und 25,6 g eines Pt/C-Katalysators (Bezugsquelle Sigma-Aldrich, 5 Gew.-% Pt bezogen auf den Kohle-Träger, 1446 $m^2/g$ BET-Oberfläche, Pt-Partikel im Bereich 1-5 nm) vorgelegt und unter Rühren mit 1000 Umdrehungen pro Minute auf 50°C erwärmt und durch Zufuhr von Sauerstoff ein Gesamtdruck von 0,3 MPa abs eingestellt. Anschließend wurden 125 g 2-Methoxyethanol, entweder vorgelegt (Beispiel 1, formal entspricht das eine Zugabe innerhalb von 0 Stunden) oder mit konstanter Rate über einen Zeitraum von 1,5 Stunden (Beispiel 2) bis 8 Stunden (Beispiel 5) zugeführt. Im Falle von Beispiel 1 (0 Stunden) wurden die 125 g 2-Methoxyethanol unverzüglich zu Beginn der Zeitmessung zugeführt. Durch eine druckgeregelte Zufuhr von Sauerstoff wurde jeweils über die gesamte Reaktionszeit hinweg der Gesamtdruck im Reaktionskalorimeter auf 0,3 MPa abs gehalten. Über die druckgeregelte Zufuhr wurde gleichzeitig der Verbrauch an Sauerstoff erfasst und somit indirekt der Umsatz an 2-Methoxyethanol über den Reaktionsverlauf hinweg bestimmt. Parallel dazu wurde die jeweils aktuell produzierte Wärmemenge erfasst. Nach dem Ende der 2-Methoxyethanol-Zugabe wurde das Reaktionskalorimeter unter den eingestellten Bedingungen bis zum Erreichen eines 2-Methoxyethanol-Umsatzes von jeweils 95% belassen. Anschließend wurde das Reaktionskalorimeter auf Raumtemperatur abgekühlt, auf Atmosphärendruck entspannt und das entnommene Reaktionsgemisch durch Filtration vom Katalysator befreit.

Das filtrierte Reaktionsgemisch wurde anschließend zur Entfernung von Wasser und nicht umgesetztem 2-Methoxyethanol in einem kontinuierlich betriebenen Sambay-Verdampfer mit einer Oberfläche von 0,046 $m^2$ bei 50°C, 25 hPa abs und mit einer Zugaberate von 1 mL Reaktionsgemisch pro Minute aufgereinigt. Das aufgereinigte Sumpfprodukt wurde entnommen und ohne weitere destillative Reinigung mittels quantitativer Gaschromatografie unter Einsatz von 1,4-Dioxan als internen Standard analysiert sowie die Farbzahl nach APHA bestimmt. Das Produkt enthielt in allen Beispielen jeweils maximal 0,3 Gew.-% Wasser und maximal 0,3 Gew.-% 2-Methoxyethanol. Alle weiteren erhaltenen Ergebnisse sind in Tabelle 1 dargestellt.

Der Wert CA berechnet sich dabei aus der insgesamt eingesetzten Masse an 2-Methoxyethanol und der insgesamt eingesetzten Masse an Wasser. Der Wert CA ist somit bei allen fünf Beispielen gleich.

Das Maximum von CR wurde aus dem zeitlichen Verlauf des Umsatzes an 2-Methoxyethanol berechnet, wobei hierzu natürlich die Stöchiometrie der Reaktion berücksichtigt wurde.

Die Beispiele zeigen, dass die Bildung des unerwünschten Methoxyessigsäure-2-methoxyethylesters mit sinkendem Maximum von CR abnimmt. Auch die Farbzahl nach APHA verringert sich mit sinkendem Maximum von CR. Sowohl der Wert CA, und somit die Massen an insgesamt zugegeben 2-Methoxyethanol und Wasser, als auch das maximal erforderliche Reaktorvolumen waren bei allen fünf Beispielen gleich. Bei gleicher Größe des Reaktionsansatzes und identischem Reaktorvolumen konnte durch die erfindungsgemäße Zugabe von 2-Methoxyethanol ein deutlich reineres Reaktionsgemisch erhalten werden. Im Vergleichsbeispiel 1 nach dem Stand der Technik enthielt das Reaktionsgemisch 2,6 Gew.-% an unerwünschtem Methoxyessigsäure-2-methoxyethylester, im erfindungsgemäßen Beispiel 3 beispielsweise nur noch 0,9 Gew.-% und im erfindungsgemäßen Beispiel 5 sogar nur noch 0,5 Gew.-%.

Entsprechend des sinkenden Maximums von CR nimmt auch der Höchstwert der produzierten Wärmemenge deutlich ab. So betrug dieser im Vergleichsbeispiel 1 sehr hohe 457 W/kg, im erfindungsgemäßen Beispiel 3 beispielsweise nur noch 81 W/Kg und im erfindungsgemäßen Beispiel 5 sogar nur noch geringe 30 W/Kg. Entsprechend dieser deutlichen Abnahme der produzierten Wärmemenge reicht beim erfindungsgemäßen Verfahren auch eine deutlich niedrigere Kühlleistung aus, welche einen kleineren Kühler mit einer deutlich niedrigeren Spitzenkühlleistung ermöglicht.

Beispiel 6

Beispiel 3 wurde wiederholt, wobei in Beispiel 6 als Reaktor ein 1,5 Liter Rührkessen (CSTR) mit Hohlwellen-Begasungsrührer verwendet wurde, deren Bodenablass mit einem Sinterglasfilter versehen war, um bei der Entleerung des Reaktors den Katalysator jeweils im Reaktor zu belassen. Nach dem Ende des Versuchs, das heißt nach Erreichen eines 2-Methoxyethanol-Umsatzes von 95%, wurde das Reaktionsgemisch durch den Sinterglasfilter entnommen und wie in Beispiel 3 gaschromatografisch analysiert sowie die Farbzahl nach APHA bestimmt. Der im Reaktor verbliebene Katalysator wurde im nächsten Ansatz unter den Bedingungen von Beispiel 3 wiederverwendet. Insgesamt wurden 20 derartiger Ansätze mit der gleiche Katalysatorcharge durchgeführt. Aktivität und Selektivität blieben im Rahmen der Meßgenauigkeit konstant.

Beispiel 6 zeigt, dass beim erfindungsgemäßen Verfahren der Katalysator über viele halbkontinuierliche Cyclen

hinweg wiederverwendet werden kann und selbst nach 20 Cyclen weder ein Verlust an Aktivität noch an Selektivität nachgewiesen werden kann.

Tabelle 1

| Beispiel | Einheit | 1 (Vergleich) | 2 (Vergleich) | 3 (Erfindung) | 4 (Erfindung) | 5 (Erfindung) |
|---|---|---|---|---|---|---|
| Zugabedauer 2ME#1 | [h] | 0 | 1,5 | 3 | 5 | 8 |
| Reaktionszeit bis Erreichen von 95% 2ME-Umsatz #1 | [h] | 6,7 | 8 | 10 | 11,7 | 13,2 |
| CA | [g/g] | 0,25 | 0,25 | 0,25 | 0,25 | 0,25 |
| Maximale Konzentration an 2ME #1 im Reaktionsgemisch | [Gew.-%] | 25,0 | 20,7 | 17,2 | 13,9 | 11,7 |
| Maximum von CR | [g/g] | 0,250 | 0,215 | 0,188 | 0,158 | 0,136 |
| Maximum von (CR · 100)/CA | [%] | 100 | 86,0 | 75,2 | 63,2 | 54,4 |
| Höchstwert der während der Umsetzung produzierten Wärmeleistung | [W/kg] | 457 | 252 | 81 | 34 | 30 |
| Gehalt an ME2MEE #3 bezogen auf den Gehalt an 2MAA#2 in aufgereinigtem Reaktionsgemisch | [Gew.-%] | 2,6 | 1,3 | 0,9 | 0,7 | 0,5 |
| Farbzahl (APHA) in aufgereinigtem Reaktionsgemisch | | > 60 | > 60 | < 50 | < 50 | < 50 |
| #1 2ME = 2-Methoxyethanol<br>#2 2MAA = 2-Methoxyessigsäure<br>#3 ME2MEE = Methoxyessigsäure-2-methoxyethylester | | | | | | |

## Patentansprüche

1. Verfahren zur Herstellung von 2-Methoxyessigsäure durch Oxidation von 2-Methoxyethanol in einer Reaktionsvorrichtung mit Sauerstoff bei einer Temperatur von 20 bis 100°C und einem Sauerstoff-Partialdruck von 0,01 bis 2 MPa in Gegenwart von Wasser und eines heterogenen Katalysators enthaltend Platin, **dadurch gekennzeichnet, dass** man das Verfahren halbkontinuierlich oder kontinuierlich durchführt, und man die Zugabe von 2-Methoxyethanol zur Reaktionsvorrichtung zeitlich und räumlich so wählt, dass in der 2-Methoxyethanol und 2-Methoxyessigsäure enthaltenden Flüssigphase in der Reaktionsvorrichtung der Quotient CR/CA zeitlich und räumlich stets ≤ 0,80 beträgt, wobei CR definiert ist als

$$CR = \frac{C(2Methoxyethanol\ Reaktor)}{C(2Methoxyethanol\ Reaktor) + C(Wasser\ Reaktor)},$$

und darin C(2Methoxyethanol Reaktor) für die Masse an 2-Methoxyethanol pro Volumenelement der 2-Methoxyethanol und 2-Methoxyessigsäure enthaltenden Flüssigphase, und C(Wasser Reaktor) für die Masse an Wasser pro Volumenelement der 2-Methoxyethanol und 2-Methoxyessigsäure enthaltenden Flüssigphase steht,
beim halbkontinuierlichen Verfahren CA definiert ist als

$$CA = \frac{MT(2Methoxyethanol\ Gesamtmasse)}{MT(2Methoxyethanol\ Gesamtmasse) + MT(Wasser\ Gesamtmasse)},$$

wobei MT(2Methoxyethanol Gesamtmasse) für die insgesamt beim halbkontinuierlichen Verfahren eingesetzte Masse an 2-Methoxyethanol, und MT(Wasser Gesamtmasse) für die insgesamt beim halbkontinuierlichen Verfahren eingesetzte Masse an Wasser steht, und
beim kontinuierlichen Verfahren CA definiert ist als

$$CA = \frac{MF(2Methoxyethanol\ Massenstrom)}{MF(2Methoxyethanol\ Massenstrom) + MF(Wasser\ Massenstrom)},$$

wobei MF(2Methoxyethanol Massenstrom) für den, der Reaktionsvorrichtung zugeführten Massenstrom an 2-Methoxyethanol, und MF(Wasser Massenstrom) für den, der Reaktionsvorrichtung zugeführten Massenstrom an Wasser steht.

**2.** Verfahren nach Anspruch 1, **dadurch gekennzeichnet, dass** man die Zugabe von 2-Methoxyethanol zur Reaktionsvorrichtung zeitlich und räumlich so wählt, dass in der 2-Methoxyethanol und 2-Methoxyessigsäure enthaltenden Flüssigphase in der Reaktionsvorrichtung der Quotient CR/CA zeitlich und räumlich stets ≤ 0,70 beträgt.

**3.** Verfahren nach den Ansprüchen 1 bis 2, **dadurch gekennzeichnet, dass** man ein Gewichtsverhältnis von Wasser zu 2-Methoxyethanol von 1 bis 5 einsetzt, wobei dieses beim halbkontinuierlichen Verfahren auf die insgesamt eingesetzten Massen an Wasser und 2-Methoxyethanol sowie beim kontinuierlichen Verfahren auf die der Reaktionsvorrichtung zugeführten Massenströme an Wasser und 2-Methoxyethanol bezogen ist.

**4.** Verfahren nach den Ansprüchen 1 bis 3, **dadurch gekennzeichnet, dass** man einen heterogenen Katalysator enthaltend 0,1 bis 10 Gew.-% Platin auf Kohle einsetzt.

**5.** Verfahren nach den Ansprüchen 1 bis 4, **dadurch gekennzeichnet, dass** man das Verfahren halbkontinuierlich durchführt und 2-Methoxyethanol über einen Zeitraum von 1 bis 10 Stunden der Reaktionsvorrichtung zuführt.

**6.** Verfahren nach den Ansprüchen 1 bis 4, **dadurch gekennzeichnet, dass** man das Verfahren kontinuierlich durchführt und man 2-Methoxyethanol und Wasser der Reaktionsvorrichtung derart zuführt, dass der Massenstrom an 2-Methoxyethanol und Wasser bezogen auf das gesamte Reaktorvolumen in der Reaktionsvorrichtung 0,05 bis 0,5 pro Stunde beträgt.

**7.** Verfahren nach den Ansprüchen 1 bis 6, **dadurch gekennzeichnet, dass** man 80 bis 99% des eingesetzten 2-Methoxyethanols umsetzt.

**8.** Verfahren nach den Ansprüchen 1 bis 7, **dadurch gekennzeichnet, dass** man das Verfahren halbkontinuierlich durchführt und die Reaktionsvorrichtung einen Reaktor aus der Gruppe Rührkessel, Rieselbettreaktor und Blasensäulenreaktor enthält.

**9.** Verfahren nach den Ansprüchen 1 bis 7, **dadurch gekennzeichnet, dass** man das Verfahren kontinuierlich durchführt und die Reaktionsvorrichtung einen Reaktor aus der Gruppe Rührkesselkaskade, Rieselbettreaktor-Kaskade, kaskadierter Blasensäulenreaktor und kaskadierter Strahlschlaufenreaktor enthält.

**10.** Verfahren nach den Ansprüchen 1 bis 9, **dadurch gekennzeichnet, dass** man aus dem erhaltenen Reaktionsgemisch die Leichtsieder Wasser und 2-Methoxyethanol durch Verdampfung entfernt.

**Claims**

**1.** A method for producing 2-methoxyacetic acid by oxidizing 2-methoxyethanol in a reaction device using oxygen at a temperature of 20 to 100°C and an oxygen partial pressure of 0.01 to 2 MPa in the presence of water and a heterogeneous catalyst containing platinum, wherein the method is carried out semi-continuously or continuously and wherein the addition of 2-methoxyethanol to the reaction device is temporally and spatially selected such that temporally and spatially, in the liquid phase containing 2-methoxyethanol and 2-methoxyacetic acid in the reaction device, the quotient of CR/CA is constantly ≤ 0.80,
wherein CR is defined as

$$CR = \frac{C(2\text{−}methoxyethanol\ reactor)}{C(2\text{−}methoxyethanol\ reactor) + C(water\ reactor)},$$

and wherein C(2-methoxyethanol reactor) denotes the mass of 2-methoxyethanol per volume element of the liquid phase containing 2-methoxyethanol and 2-methoxyacetic acid, and C(water reactor) denotes the mass of water per volume element of the liquid phase containing 2-methoxyethanol and 2-methoxyacetic acid,
in the semi-continuous method, CA is defined as

$$CA = \frac{MT(2\text{−}methoxyethanol\ total\ mass)}{MT(2\text{−}methoxyethanol\ total\ mass) + MT(water\ total\ mass)},$$

wherein MT(2-methoxyethanol total mass) denotes the total mass of 2-methoxyethanol used in the semi-continuous method, and MT(water total mass) denotes the total mass of water used in the semi-continuous method, and
in the continuous method, CA is defined as

$$CA = \frac{MF(2\text{−}methoxyethanol\ mass\ flow)}{MF(2\text{−}methoxyethanol\ mass\ flow) + MF(water\ mass\ flow)},$$

wherein MF(2-methoxyethanol mass flow) denotes the mass flow of 2-methoxyethanol supplied to the reaction device, and MF(water mass flow) denotes the mass flow of water supplied to the reaction device.

**2.** The method according to claim 1, wherein the addition of 2-methoxyethanol to the reaction device is temporally and spatially selected such that temporally and spatially, in the liquid phase containing 2-methoxyethanol and 2-methoxyacetic acid in the reaction device, the quotient of CR/CA is constantly $\leq 0.70$.

**3.** The method according to claims 1 and 2, wherein a ratio by weight of water to 2-methoxyethanol of 1 to 5 is used, wherein this is based in the semi-continuous method on the total masses of water and 2-methoxyethanol used and in the continuous method on the mass flows of water and 2-methoxyethanol supplied to the reaction device.

**4.** The method according to claims 1 to 3, wherein a heterogeneous catalyst containing 0.1 to 10 wt% of platinum on carbon is used.

**5.** The method according to claims 1 to 4, wherein the method is carried out semi-continuously, and 2-methoxyethanol is supplied to the reaction device over a period of 1 to 10 h.

**6.** The method according to claims 1 to 4, wherein the method is carried out continuously and 2-methoxyethanol and water are supplied to the reaction device such that the mass flow of 2-methoxyethanol and water based on the total reactor volume in the reaction device is 0.05 to 0.5 per h.

**7.** The method according to claims 1 to 6, wherein 80 to 99% of the 2-methoxyethanol used is reacted.

**8.** The method according to claims 1 to 7, wherein the method is carried out semi-continuously and the reaction device comprises a reactor from the group of a stirred vessel, a trickle-bed reactor, and a bubble column reactor.

**9.** The method according to claims 1 to 7, wherein the method is carried out continuously and the reaction device comprises a reactor from the group of a stirred vessel cascade, a trickle-bed reactor cascade, a cascaded bubble column reactor, and a cascaded jet loop reactor.

**10.** The method according to claims 1 to 9, wherein the low boilers water and 2-methoxyethanol are removed by evaporation from the reaction mixture obtained.

**Revendications**

**1.** Procédé pour la préparation d’acide 2-méthoxyacétique par oxydation de 2-méthoxyéthanol dans un dispositif de

réaction avec de l'oxygène à une température de 20 à 100 °C et à une pression partielle d'oxygène de 0,01 à 2 MPa en présence d'eau et d'un catalyseur hétérogène contenant du platine, **caractérisé en ce qu'**on met en œuvre le procédé de manière semi-continue ou continue, et on choisit l'ajout de 2-méthoxyéthanol au dispositif de réaction dans le temps et dans l'espace de telle façon que, dans la phase liquide contenant du 2-méthoxyéthanol et de l'acide 2-méthoxyacétique dans le dispositif de réaction, le quotient CR/CA dans le temps et dans l'espace est toujours ≤ 0,80,
CR étant défini comme

$$CR = \frac{C(2-méthoxyéthanol\ réacteur)}{C(2-méthoxyéthanol\ réacteur) + C(eau\ réacteur)}$$

et C(2-méthoxyéthanol réacteur) représentant la masse de 2-méthoxyéthanol par élément de volume de la phase liquide contenant du 2-méthoxyéthanol et de l'acide 2-méthoxyacétique, et C(eau réacteur) représentant la masse d'eau par élément de volume de la phase liquide contenant du 2-méthoxyéthanol et de l'acide 2-méthoxyacétique, dans le procédé de manière semi-continue, CA étant défini comme

$$CA = \frac{MT(2-méthoxyéthanol\ masse\ totale)}{MT(2-méthoxyéthanol\ masse\ totale) + MT(eau\ masse\ totale)}$$

MT(2-méthoxyéthanol masse totale) représentant la masse totale de 2-méthoxyéthanol utilisée dans le procédé de manière semi-continue, et MT(eau masse totale) représentant la masse totale d'eau utilisée dans le procédé de manière semi-continue, et
dans le procédé de manière continue, CA étant défini comme

$$CA = \frac{MF(2-méthoxyéthanol\ flux\ de\ masse)}{MF(2-méthoxyéthanol\ flux\ de\ masse) + MF(eau\ flux\ de\ masse)}$$

MF(2-méthoxyéthanol flux de masse) représentant le flux de masse de 2-méthoxyéthanol alimenté au dispositif de réaction, et MF(eau flux de masse) représentant le flux de masse d'eau alimenté au dispositif de réaction.

**2.** Procédé selon la revendication 1, **caractérisé en ce que** l'ajout de 2-méthoxyéthanol au dispositif de réaction est choisi dans le temps et dans l'espace de telle manière que, dans la phase liquide contenant du 2-méthoxyéthanol et de l'acide 2-méthoxyacétique dans le dispositif de réaction, le quotient CR/CA dans le temps et dans l'espace est toujours ≤ 0,70.

**3.** Procédé selon les revendications 1 et 2, **caractérisé en ce qu'**on utilise un rapport pondéral d'eau à 2-méthoxyéthanol de 1 à 5, celui-ci, dans le procédé de manière semi-continue se rapportant aux masses totales utilisées d'eau et de 2-méthoxyéthanol, ainsi que dans le procédé de manière continue se rapportant aux flux de masse d'eau et de 2-méthoxyéthanol alimentés au dispositif de réaction.

**4.** Procédé selon les revendications 1 à 3, **caractérisé en ce qu'**on utilise un catalyseur hétérogène contenant 0,1 à 10 % en poids de platine sur charbon.

**5.** Procédé selon les revendications 1 à 4, **caractérisé en ce qu'**on met en œuvre le procédé de manière semi-continue et on alimente le 2-méthoxyéthanol sur une période de temps de 1 à 10 heures au dispositif de réaction.

**6.** Procédé selon les revendications 1 à 4, **caractérisé en ce qu'**on met en œuvre le procédé de manière continue et on alimente le 2-méthoxyéthanol et l'eau au dispositif de réaction de telle manière que le flux de masse de 2-méthoxyéthanol et d'eau, par rapport au volume total du réacteur dans le dispositif de réaction, est de 0,05 à 0,5 par heure.

**7.** Procédé selon les revendications 1 à 6, **caractérisé en ce qu'**on transforme 80 à 99 % du 2-méthoxyéthanol utilisé.

**8.** Procédé selon les revendications 1 à 7, **caractérisé en ce qu'**on met en œuvre le procédé de manière semi-continue et le dispositif de réaction contient un réacteur du groupe constitué par une cuve d'agitation, un réacteur à lit fluidisé

et un réacteur à colonnes à bulles.

**9.** Procédé selon les revendications 1 à 7, **caractérisé en ce qu'**on met en œuvre le procédé de manière continue et le dispositif de réaction contient un réacteur du groupe constitué par une cascade de cuves d'agitation, une cascade de réacteurs à lit fluidisé, un réacteur à colonnes à bulles en cascade et un réacteur en boucle à jet en cascade.

**10.** Procédé selon les revendications 1 à 9, **caractérisé en ce qu'**on retire par évaporation les composés volatiles eau et 2-méthoxyéthanol du mélange de réaction obtenu.

Fig. 1/7

a)

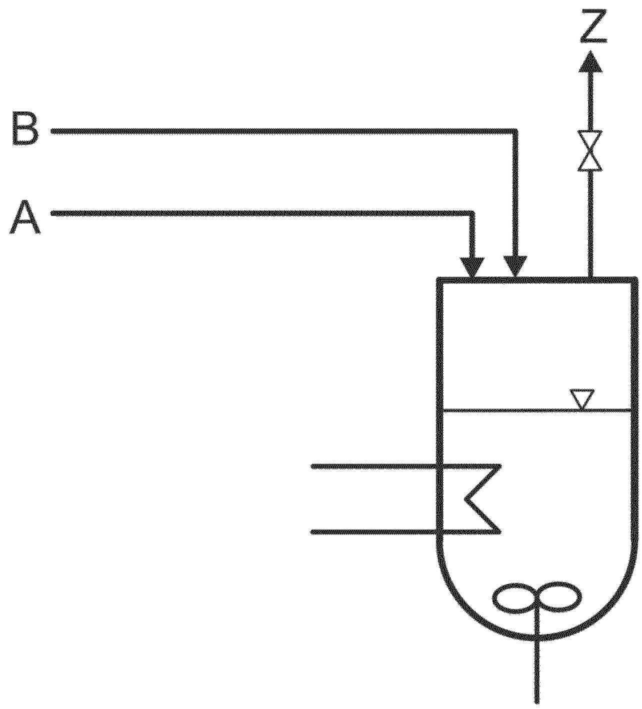


b)

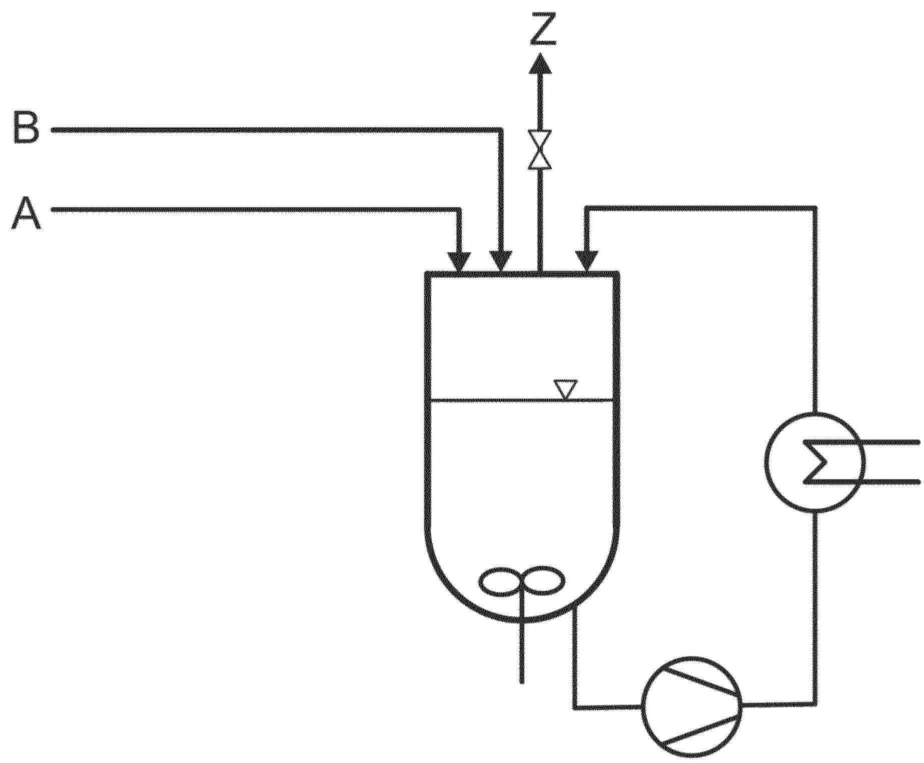

Fig. 2/7

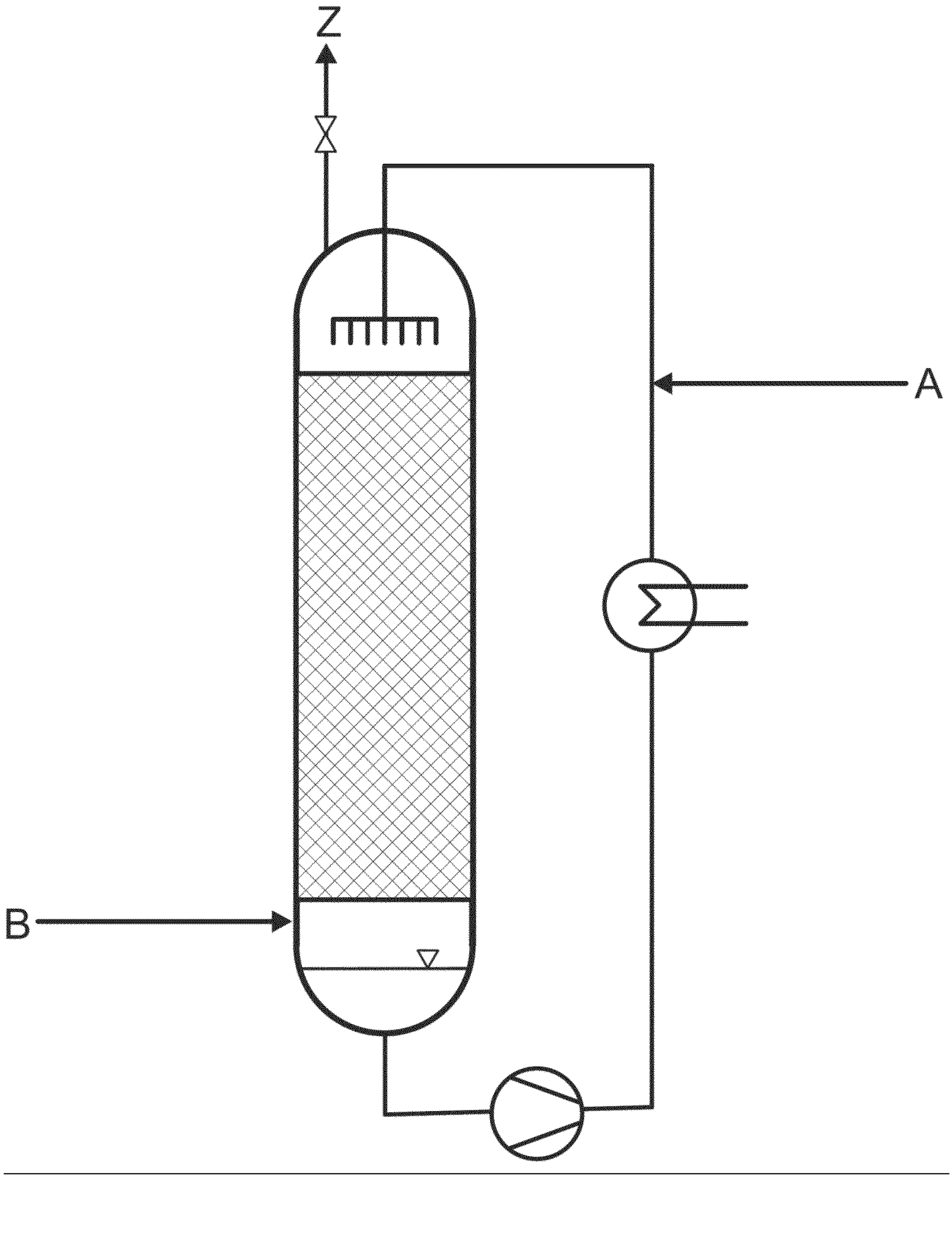

Fig. 3/7

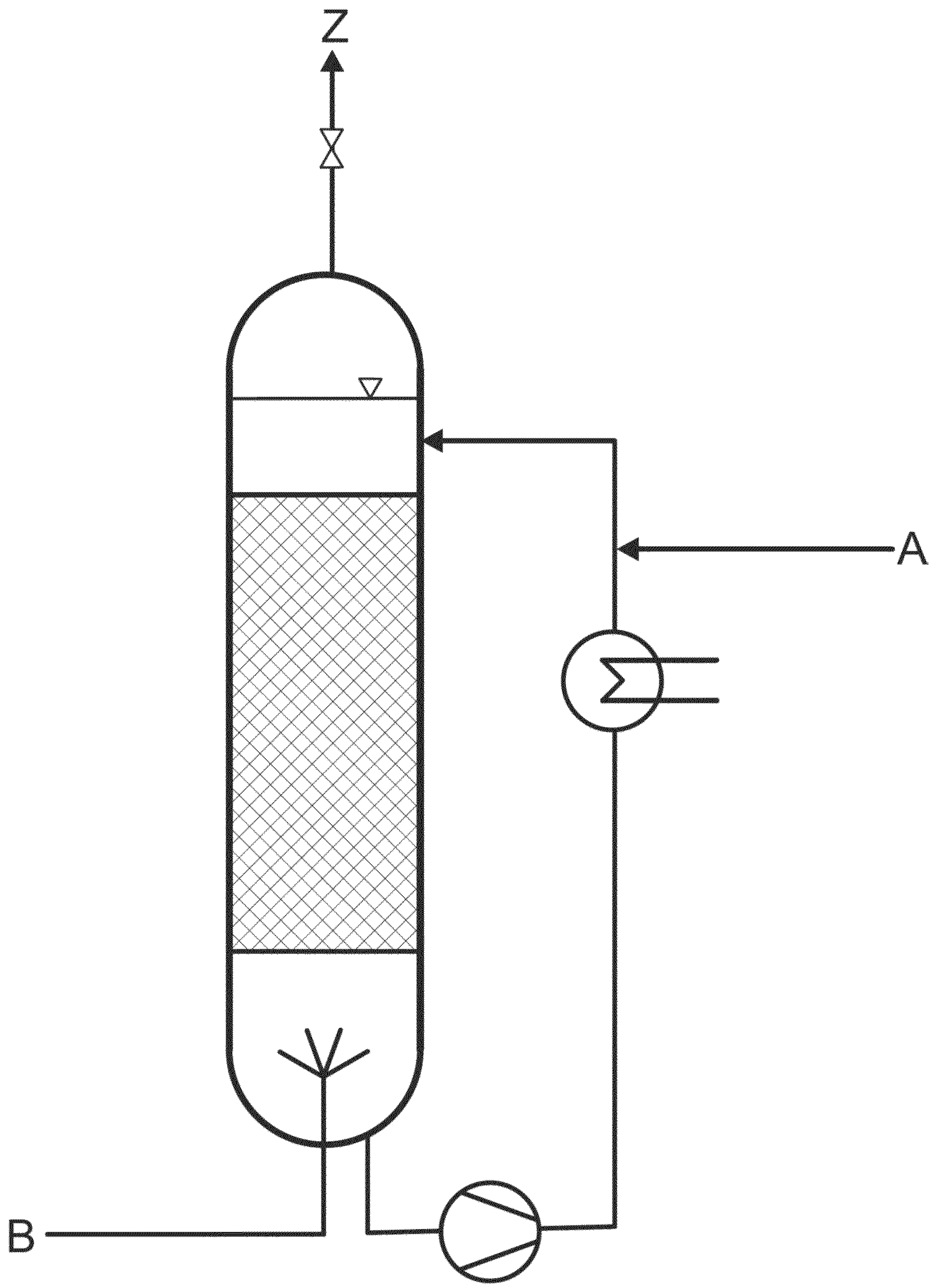

Fig. 4/7

a)

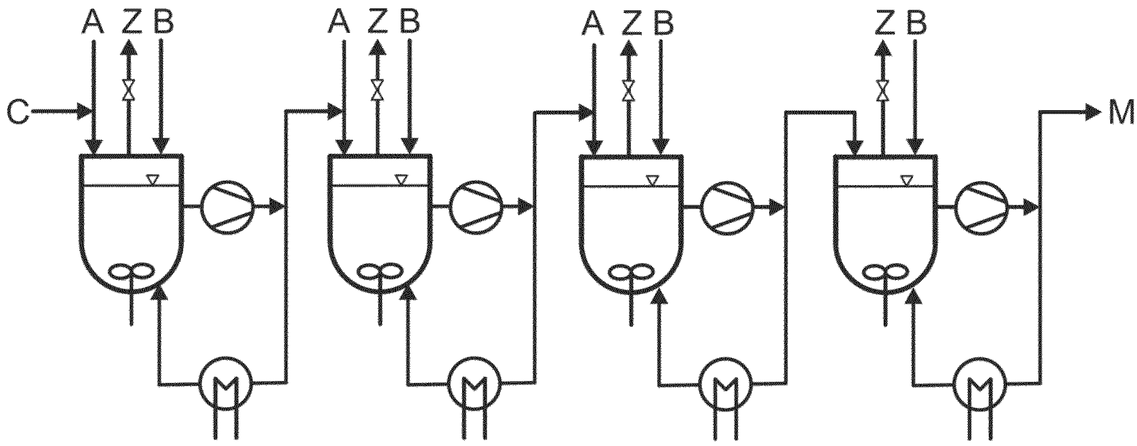


b)

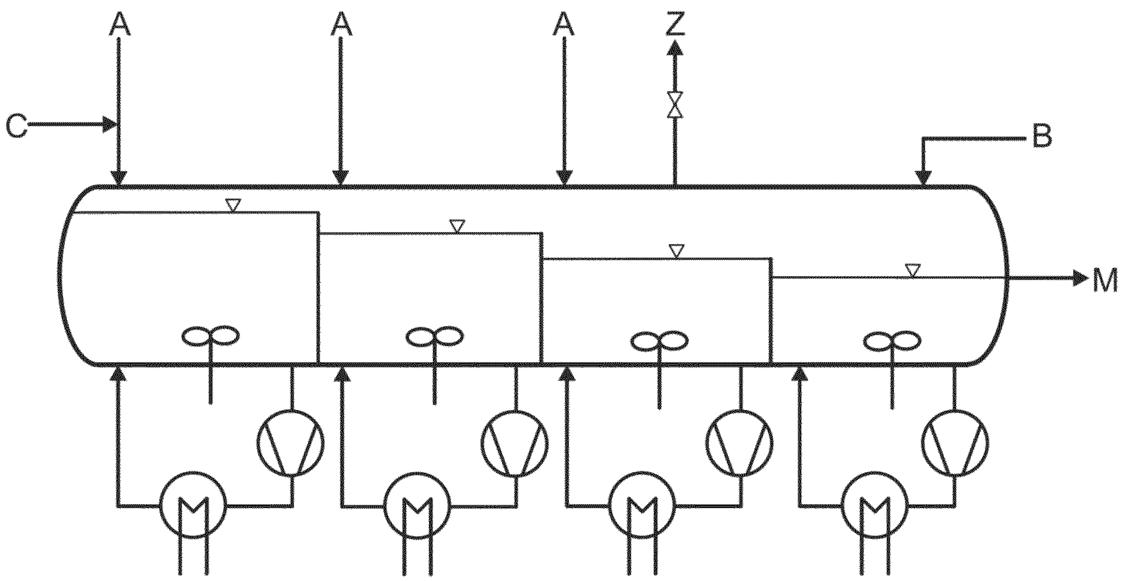

Fig. 5/7

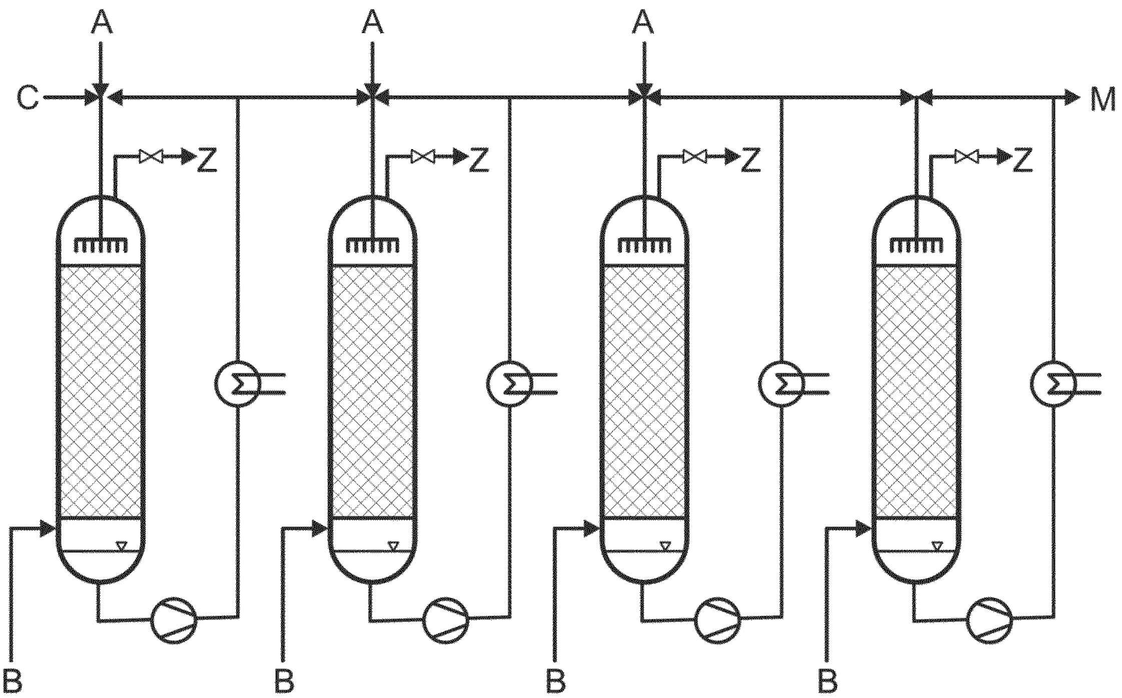

Fig. 6/7

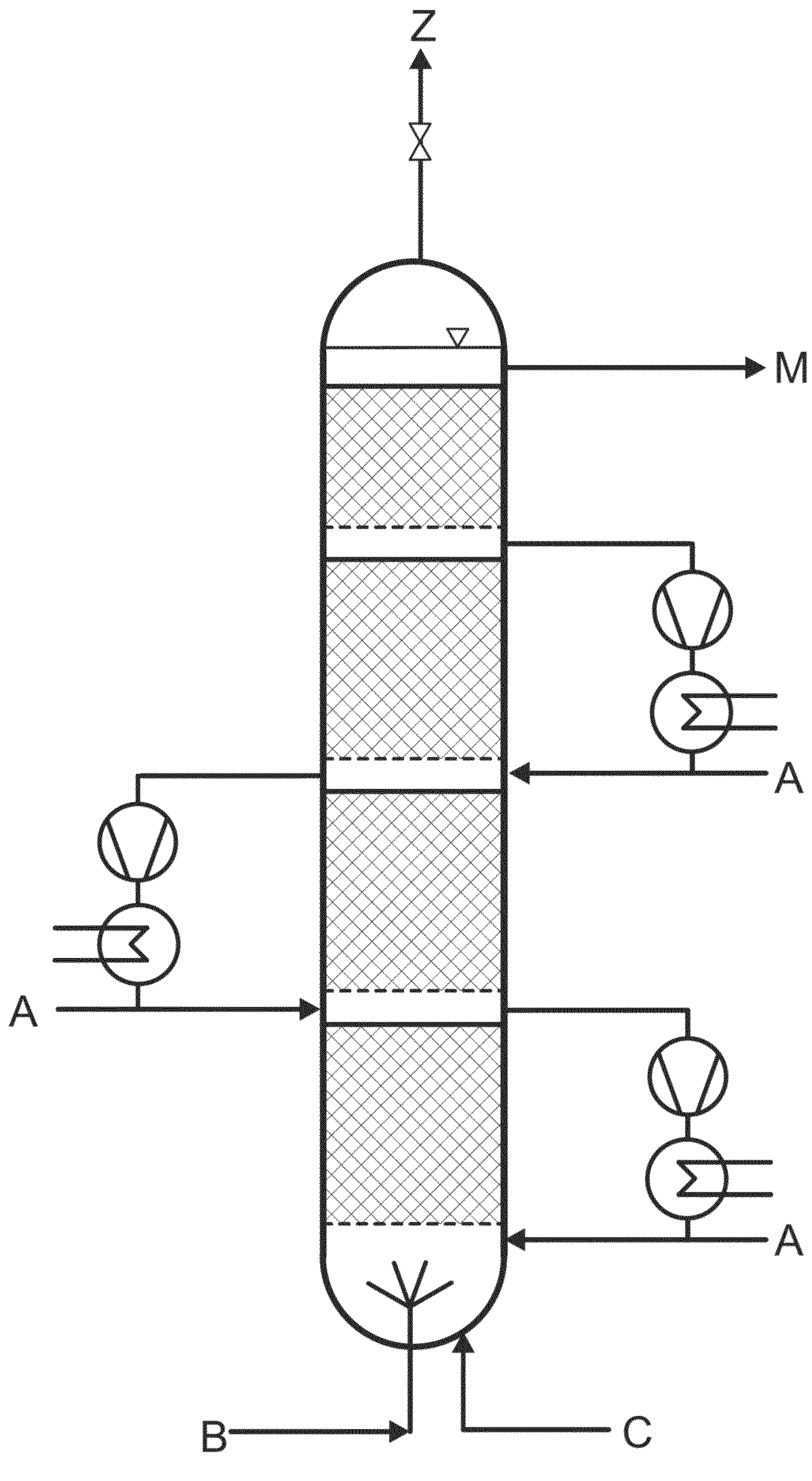

Fig. 7/7

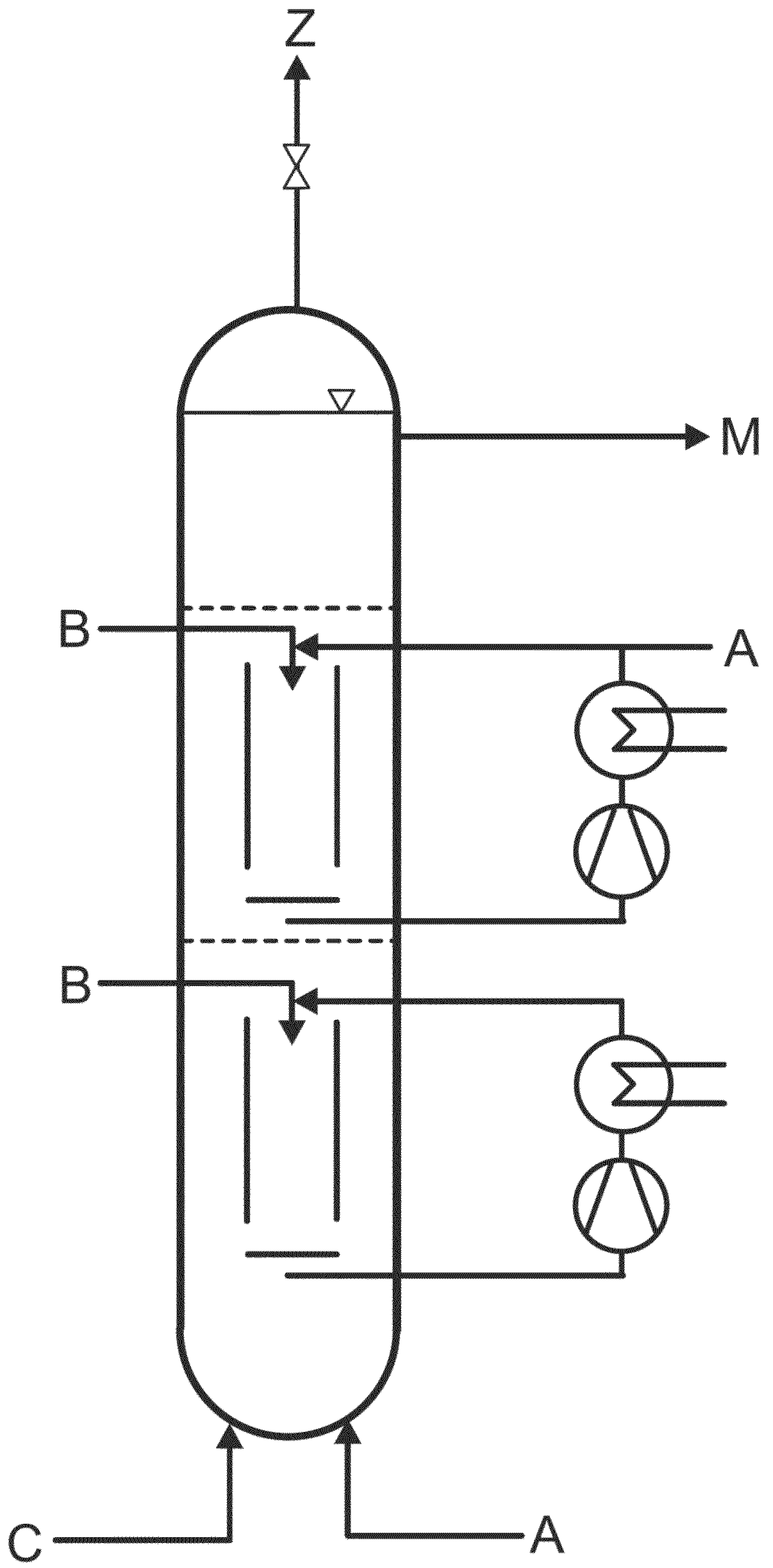

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3342858 A
- DE 2936123 A
- CN 104892390 A
- DE 3345807 A